(19) Europäisches Patentamt
European Patent Office
Office européen des brevets

(11) EP 4 065 236 B1

(12) # EUROPÄISCHE PATENTSCHRIFT

(45) Veröffentlichungstag und Bekanntmachung des Hinweises auf die Patenterteilung:
**11.06.2025 Patentblatt 2025/24**

(21) Anmeldenummer: **20780953.4**

(22) Anmeldetag: **14.09.2020**

(51) Internationale Patentklassifikation (IPC):
***A61Q 5/08*** *(2006.01)* ***A61K 8/898*** *(2006.01)*
***A61K 8/362*** *(2006.01)* ***A61K 8/365*** *(2006.01)*
***A61K 8/19*** *(2006.01)*

(52) Gemeinsame Patentklassifikation (CPC):
**A61Q 5/08; A61K 8/19; A61K 8/362; A61K 8/365; A61K 8/898**

(86) Internationale Anmeldenummer:
**PCT/EP2020/075627**

(87) Internationale Veröffentlichungsnummer:
**WO 2021/104705 (03.06.2021 Gazette 2021/22)**

(54) **VERFAHREN ZUR ENTFÄRBUNG VON KERATINISCHEM MATERIAL, DAS DURCH ANWENDUNG EINES PIGMENTS GEFÄRBT WURDE**

METHOD FOR THE DECOLORIZATION OF KERATIN MATERIAL THAT HAS BEEN DYED USING A PIGMENT

PROCÉDÉ DE DÉCOLORATION DE MATIÈRE KÉRATINIQUE COLORÉE À L’AIDE D’UN PIGMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.11.2019 DE 102019218237**

(43) Veröffentlichungstag der Anmeldung:
**05.10.2022 Patentblatt 2022/40**

(73) Patentinhaber: **Henkel AG & Co. KGaA**
**40589 Düsseldorf (DE)**

(72) Erfinder:
- **KRUCK, Constanze**
  **41515 Grevenbroich (DE)**
- **HILBIG, Sandra**
  **44894 Bochum (DE)**
- **MOCH, Melanie**
  **41542 Dormagen (DE)**
- **DICKHOF, Susanne**
  **41748 Viersen (DE)**
- **KESSLER-BECKER, Daniela**
  **51371 Leverkusen (DE)**

(56) Entgegenhaltungen:
**EP-A2- 2 168 633**
**DE-A1- 102015 222 214**
**DE-A1- 102016 209 980**
**GB-A- 2 513 319**

EP 4 065 236 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Anmeldung liegt auf dem Gebiet der Kosmetik und betrifft ein Verfahren zur Entfärbung von Keratinmaterial, welches durch Anwendung mindestens einem aminofunktionalisierten Silikonpolymer und mindestens eines Pigments gefärbt wurde. Das im Rahmen dieses Verfahrens applizierte Entfärbemittel ist gekennzeichnet durch seinen Gehalt an mindestens einem Salz eines ein- oder zweiwertigen Kations sowie einen pH-Wert, der im Bereich von 1,0 bis 4,7 liegt. Das Entfärbemittel wird auf das gefärbte Keratinmaterial aufgetragen und nach einer Einwirkzeit wieder abgespült.

Ein zweiter Gegenstand der vorliegenden Anmeldung ist ein Verfahren zum Färben und späteren Entfärben von Keratinmaterial, bei welchem zunächst ein Färbemittel enthaltend mindestens ein aminofunktionalisiertes Silikonpolymer und ein Pigment auf das Keratin aufgetragen wird, und in den nachfolgenden Schritten die Entfärbung durch Anwendung des zuvor beschriebenen Entfärbemittels vorgenommen wird.

Ein dritter Gegenstand der vorliegenden Anmeldung ist eine Mehrkomponenten-Verpackungseinheit, welche in getrennt konfektionierten Containern das Färbe- und das Entfärbemittel enthält.

Die Veränderung von Form und Farbe von keratinischen Fasern, insbesondere von Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

DE102016209980 offenbart ein Verfahren zur Entfernung von durch Direktfarbstoffe erhaltener Farbe aus dem Haar.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus. Eine nach wie vor bestehende Herausforderung ist daher die Suche nach alternativen, leistungsstarken Färbeverfahren. Ein mögliches, alternatives Färbesystem, das in letzter Zeit zunehmend in den Fokus rückt, beruht auf dem Einsatz von farbigen Pigmenten.

Die Färbung mit Pigmenten bietet verschiedene wesentliche Vorteile. Da die Pigmente sich lediglich von außen an die Keratinmaterialen, insbesondere an die Haarfasern, anlagern, ist die mit dem Färbeprozess verbundene Schädigung ganz besonders gering. In aktuellen Arbeiten wurde das Problem der geringen Haltbarkeit dieses Färbesystems adressiert. In diesem Zusammenhang konnte gefunden werden, dass die Waschechtheit der mit Pigmenten erhaltenen Farbresultate durch Kombination der Pigmente mit bestimmten aminofunktionalisierten Silikonpolymeren stark verbessert werden konnte.

Durch Einsatz eines geeigneten Entfärbemittels besteht die Möglichkeit, diese Färbungen ohne Beeinflussung der ursprünglichen Haarfarbe des Anwender wieder zu entfernen. Auf diese Weise bietet sich dem Anwender die Option, unmittelbar und ohne großen Aufwand wieder zu seiner Ursprungshaarfarbe zurückkehren zu können. Insbesondere für die Konsumenten, die ihre Haare nicht regelmäßig nachfärben möchten, ist dieser Färbeprozess daher besonders attraktiv.

Ein gut geeignetes Entfärbemittel sollte in der Lage sein, den auf der Oberfläche des Keratinmaterials durch die Anwendung von Pigmenten bzw. Pigmenten und Aminosilikonen ausgebildeten farbigen Film möglichst rückstandsfrei zu entfernen. Wird dieser gefärbte Film nur teilweise bzw. unvollständig entfernt, sind unerwünschte Farbverschiebungen bzw. ein fleckiges Farbergebnis die Folge, die vom Anwender als höchst unattraktiv empfunden werden.

Zur Erhöhung des Anwenderkomforts sollte ein Entfärbemittel die farbigen Filme innerhalb eines möglichst kurzen Zeitraums entfernen, und das Keratinmaterial bzw. die Haare sollten durch Anwendung des Entfärbemittels nicht geschädigt werden.

Aufgabe der vorliegenden Erfindung war daher die Bereitstellung eines Entfärbemittels zur Entfärbung von gefärbten keratinischen Fasern, welche zuvor durch Anwendung von mindestens einem Pigment, bzw. mindestens einem aminofunktionalisierten Silikonpolymer und einem Pigment, gefärbt wurden. Hierbei sollte die Entfärbung möglichst vollständig sein, so dass die Färbung des Keratinmaterials im Idealfall wieder der Ursprungsfarbe entspricht. Des Weiteren sollte die Entfärbung lang anhaltend und gleichmäßig sein, und die entfärbten Keratinfasern sollten weder Nuancenverschiebungen noch Ungleichmäßigkeiten im Farbergebnis erleiden. Zudem sollte das Keratinmaterial durch das Entfärbemittel möglichst wenig geschädigt werden.

Überraschenderweise hat sich nun herausgestellt, dass diese Aufgabe in vollumfänglicher Weise gelöst werden kann, wenn keratinisches Material, das zuvor mit mindestens einem aminofunktionalisierten Silikonpolymer und mit mindestens einem Pigment gefärbt wurde, mit einem Entfärbemittel behandelt wird, welches mindestens einSalz eines ein- oder zweiwertigen Kations enthält und einen pH-Wert im Bereich von 1,0 bis 4,7 besitzt.

Ein erster Gegenstand der vorliegenden Erfindung ist ein Verfahren zur Entfärbung von Keratinmaterial, welches durch Anwendung von mindestens einem Pigment gefärbt wurde, wobei ein Entfärbemittel, welches

(a) mindestens ein Salz eines ein- oder zweiwertigen Kations enthält, und
(b) einen pH-Wert von 1,0 bis 4,7 besitzt,

auf das gefärbte Keratinmaterial aufgetragen und nach einer Einwirkzeit wieder abgespült wird.

Die zu dieser Erfindung führenden Arbeiten haben gezeigt, dass Keratinfasern, insbesondere Haare, durch Anwendung von Pigmenten intensiv gefärbt werden konnten. Ganz besonders intensive Färbeergebnisse wurden hierbei erhalten, wenn die Färbung mit einer Kombination aus Pigment und Aminosilikon erfolgte. Das Pigment bzw. das Gemisch aus Pigment und Aminosilikon lagerte sich in Form eines farbigen Films auf der Oberfläche der Keratinfasern ab. Weiterhin hat sich gezeigt, dass diese Färbungen durch Anwendung des zuvor beschriebenen Entfärbemittels vollständig innerhalb eines kurzen Anwendungszeitraums wieder entfärbt werden konnten, ohne die Haare zu schädigen.

Entfärbung von keratinischem Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

Unter dem Begriff "Mittel zur Entfärbung" wird im Rahmen dieser Erfindung verstanden, dass auf dem Keratinmaterial eine Färbung, die durch Anwendung von mindestens einem Pigment, bzw. mindestens einem aminofunktionalisierten Silikonpolymer und einem Pigment erzeugt wurde, wieder entfernt werden kann. Bei dieser Färbung wird das Keratinmaterial bzw. die Keratinfaser von einem gefärbten Film umhüllt, der aus den Pigment bzw. dem Pigment und Aminosilikon ausgebildet wird. Die Anwendung des Entfärbemittels erfolgt erfindungsgemäß nach der Anwendung des Färbemittels und ist in der Lage, diesen gefärbten Film wieder von dem Keratinmaterial zu entfernen.

Kennzeichnend für das erfindungsgemäße Verfahren ist die Anwendung des Entfärbemittels auf Keratinmaterial, welches zuvor durch Anwendung von mindestens einem Pigment, bzw. mindestens einem Aminosilikon und einem Pigment, gefärbt wurde.

aminofunktionalisiertes Silikonpolymer im Färbemittel

Das im erfindungsgemäßen Verfahren eingesetzte Entfärbemittel zeigte eine besonders starke Wirkung, wenn bei der vorangehenden Färbung des Keratinmaterials bzw. der Keratinfasern eine Kombination aus Pigmenten mit Aminosilikonen eingesetzt wurden.

Im Rahmen einer ganz besonders bevorzugten Ausführungsform ist ein erfindungsgemäßes Verfahren daher dadurch gekennzeichnet, dass das Entfärbemittel auf Keratinmaterial aufgetragen wird, welches durch Anwendung von mindestens einem aminofunktionalisierten Silikonpolymer und mindestens einem Pigment gefärbt wurde.

Das aminofunktionalisiertes Silikonpolymer kann alternativ auch als Aminosilikon oder Amodimethicone bezeichnet werden.

Silikonpolymere sind im allgemeinen Makromoleküle mit einem Molekulargewicht von mindestens 500 g/mol, bevorzugt mindestens 1000 g/mol, weiter bevorzugt von mindestens 2500 g/mol, besonders bevorzugt von mindestens 5000 g/mol, welche sich wiederholende organische Einheiten umfassen.

Das maximale Molekulargewicht des Silikonpolymers hängt von dem Polymerisationsgrad (Anzahl der polymerisierten Monomere) und der Ansatzgröße ab und wird auch durch die Polymerisationsmethode mitbestimmt. Im Sinne der vorliegenden Erfindung ist es bevorzugt, wenn das maximale Molekulargewicht des Silikonpolymers nicht mehr als

$10^7$ g/mol, bevorzugt nicht mehr als $10^6$ g/mol und besonders bevorzugt nicht mehr als $10^5$ g/mol beträgt.

Die Silikonpolymere umfassen viele Si-O-Wiederholungseinheiten, wobei die Si-Atome organische Reste wie beispielsweise Alkylgruppen oder substituierte Alkylgruppen tragen können. Alternativ wird ein Silikonpolymer daher auch als Polydimethylsiloxan bezeichnet.

In Entsprechung des hohen Molekulargewichts der Silikonpolymere basieren diese auf mehr als 10 Si-O Wiederholungseinheiten, bevorzugt mehr als 50 Si-O-Wiederholungseinheiten und besonders bevorzugt mehr als 100 Si-O-Wiederholungseinheiten, ganz besonders bevorzugt mehr als 500 Si-O-Wiederholungseinheiten.

Unter einem aminofunktionalisierten Silikonpolymer wird ein funktionalisiertes Silikon verstanden, welches mindestens eine Struktureinheit mit einer Aminogruppe trägt. Bevorzugt trägt das aminofunktionalisierte Silikonpolymer mehrere Struktureinheiten mit jeweils mindestens einer Aminogruppe. Unter einer Aminogruppe wird eine primäre Aminogruppe, eine sekundäre Aminogruppe und eine tertiäre Aminogruppe verstanden. Alle diese Aminogruppen können im sauren Milieu protoniert werden und liegen dann in ihrer kationischen Form vor.

Prinzipiell konnte eine gute Färbeleistung mit aminofunktionalisierten Silikonpolymeren erzielt werden, wenn diese mindestens eine primäre, mindestens eine sekundäre und/oder mindestens eine tertiäre Aminogruppe tragen. Intensive Färbungen mit der besten Waschechtheit wurden jedoch erhalten, wenn ein aminofunktionalisiertes Silikonpolymer im Mittel eingesetzt wurde, welches mindestens eine sekundäre Aminogruppe enthält.

In einer ganz besonders bevorzugten Ausführungsform ist ein erfindungsgemäßes Verfahren dadurch gekennzeichnet, dass das Entfärbemittel auf Keratinmaterial aufgetragen wird, welches durch Anwendung von mindestens einem aminofunktionalisierten Silikonpolymer mit mindestens einer sekundären Aminogruppe gefärbt wurde.

Die sekundäre(n) Aminogruppe(n) kann bzw. können sich an verschiedenen Positionen des aminofunktionalisierten Silikonpolymers befinden. Ganz besonders gute Farbresultate wurden erhalten, wenn ein aminofunktionalisiertes Silikonpolymer eingesetzt wurde, dass mindestens eine, bevorzugt mehrere Struktureinheiten der Formel (Si-Amino) besitzt.

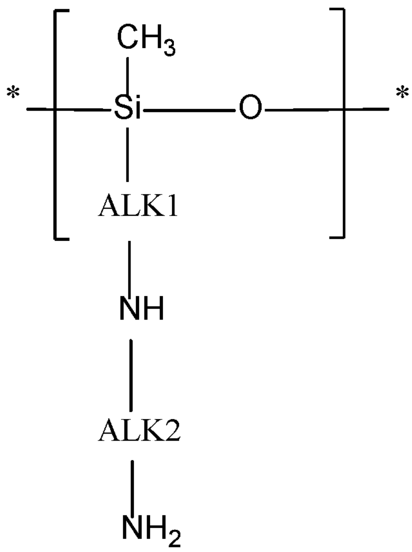

(Si-Amino)

In den Struktureinheiten der Formel (Si-Amino) stehen die Kürzel ALK1 und ALK2 unabhängig voneinander für eine lineare oder verzweigte, zweiwertige $C_1$-$C_{20}$-Alkylengruppe.

In einer weiteren ganz besonders bevorzugten Ausführungsform ist ein erfindungsgemäßes Verfahren dadurch gekennzeichnet, dass das Entfärbemittel auf Keratinmaterial aufgetragen wird, welches durch Anwendung von mindestens einem aminofunktionalisierten Silikonpolymer gefärbt wurde, welches mindestens eine Struktureinheit der Formel (Si-Amino) umfasst,

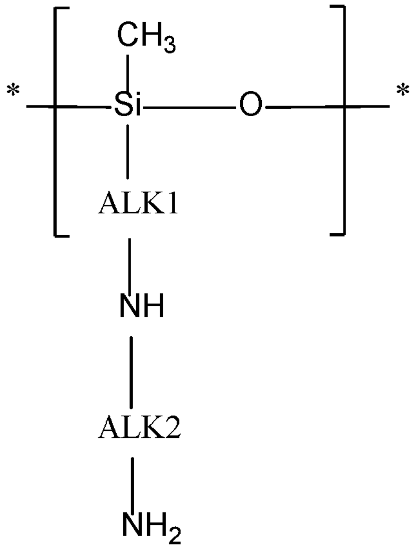

(Si-Amino)

wobei
ALK1 und ALK2 unabhängig voneinander für eine lineare oder verzweigte, zweiwertige $C_1$-$C_{20}$-Alkylengruppe stehen.

Die mit einem Stern (*) gekennzeichneten Positionen geben hierbei jeweils die Bindung zu weiteren Struktureinheiten des Silikonpolymers an. Beispielsweise kann das dem Stern benachbarte Silicium-Atom an ein weiteres Sauerstoffatom gebunden sein, und das dem Stern benachbarte Sauerstoffatom kann an ein weiteres Siliciumatom oder auch an eine $C_1$-$C_6$-Alkylgruppe gebunden sein.

Eine zweiwertige $C_1$-$C_{20}$-Alkylengruppe kann alternativ auch als eine divalente oder zweibindige $C_1$-$C_{20}$-Alkylengruppe bezeichnet werden, womit gemeint ist, dass jede Gruppierung ALK1 bzw. AK2 zwei Bindungen eingehen kann.

Im Fall von ALK1 erfolgt eine Bindung vom Silicium-Atom zur Gruppierung ALK1, und die zweite Bindung besteht zwischen ALK1 und der sekundären Aminogruppe.

Im Fall von ALK2 erfolgt eine Bindung von der sekundären Aminogruppe zur Gruppierung ALK2, und die zweite Bindung besteht zwischen ALK2 und der primären Aminogruppe.

Beispiele für eine lineare zweiwertige $C_1$-$C_{20}$-Alkylengruppe sind beispielsweise die Methylen-gruppe ($-CH_2-$), die Ethylengruppe ($-CH_2-CH_2-$), die Propylengruppe ($-CH_2-CH_2-CH_2-$) und die Butylengruppe ($-CH_2-CH_2-CH_2-CH_2-$). Die Propylengruppe ($-CH_2-CH_2-CH_2-$) ist besonders bevorzugt. Ab einer Kettenlänge von 3 C-Atomen können zweiwertige Alkylengruppen auch verzweigt sein. Beispiele für verzweigte, zweiwertige $C_3$-$C_{20}$-Alkylengruppen sind ($-CH_2-CH(CH_3)-$) und ($-CH_2-CH(CH_3)-CH_2-$).

In einer weiteren besonders bevorzugten Ausführungsform stellen die Struktureinheiten der Formel (Si-Amino) Wiederholungseinheiten im aminofunktionalisierten Silikonpolymer dar, so dass das Silikonpolymer mehrere Struktureinheiten der Formel (Si-Amino) umfasst.

Im Folgenden werden besonders gut geeignete aminofunktionalisierte Silikonpolymere mit mindestens einer sekundären Aminogruppe aufgelistet.

Färbungen mit den allerbesten Waschechtheiten konnten erhalten werden, wenn bei der vorangehenden Färbung mindestens ein Mittel auf dem keratinischen Material appliziert wurde, das mindestens ein aminofunktionalisiertes Silikonpolymer enthält, das Struktureinheiten der Formel (Si-I) und der Formel (Si-II) umfasst

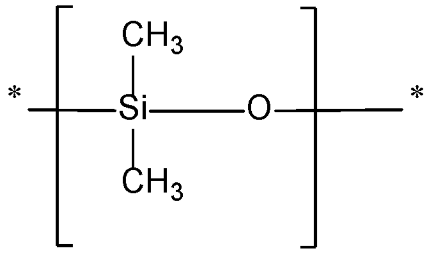

(Si-I)

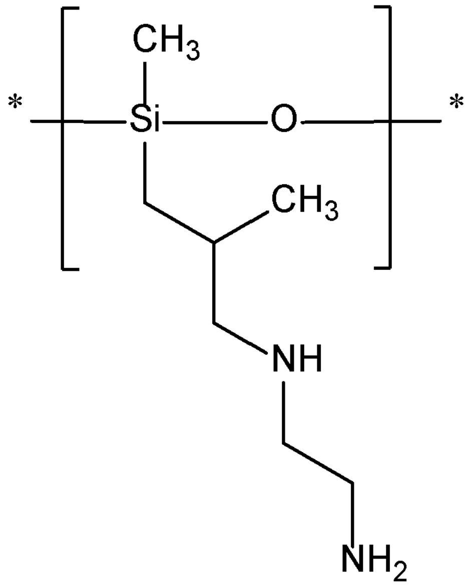

(Si-II).

In einer weiteren explizit ganz besonders bevorzugten Ausführungsform ist ein erfindungsgemäßes Verfahren dadurch gekennzeichnet, dass das Entfärbemittel auf Keratinmaterial aufgetragen wird, welches durch Anwendung von mindestens einem aminofunktionalisierten Silikonpolymer gefärbt wurde,welches Struktureinheiten der Formel (Si-I) und der Formel (Si-II) umfasst

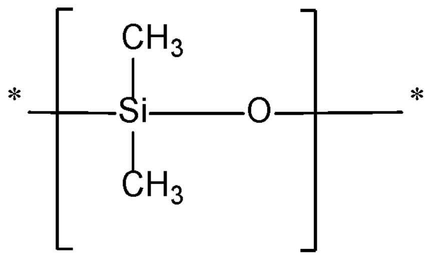

(Si-I)

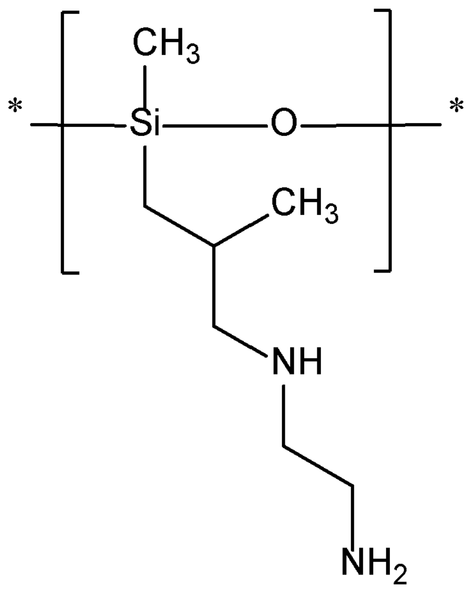

(Si-II).

Ein entsprechendes aminofunkionalisiertes Silikonpolymer mit den Struktureinheiten (Si-I) und (Si-II) ist beispielweise das Handelsprodukt DC 2-8566 bzw. Dowsil 2-8566 Amino Fluid, das von der Firma Dow Chemical Company komerziell vertrieben wird und welches die Benennung "Siloxanes and Silicones, 3-[(2-Aminoethyl)amino]-2-methylpropyl Me, Di-Me-Siloxane" sowie die CAS-Nummer 106842-44-8 trägt. Ein weiteres besonders bevorzugtes Handelsprodukt ist Dowsil AP-8658 Amino Fluid, das ebenfalls von der Firma Dow Chemical Company kommerziell vertrieben wird.

Im Rahmen einer weiteren bevorzugten Ausführungsform kann die Anwendung des Entfärbemittel auch auf Keratinmaterial erfolgen, welches zuvor durch die Anwendung eines Färbemittels gefärbt wurde, das mindestens ein aminofunktionelles Silikonpolymer der Formel der Formel (Si-III) enthält,

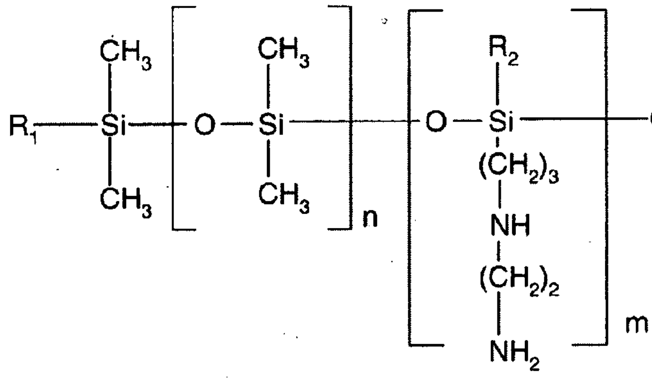

(Si-III)

wobei

- m und n bedeuten Zahlen, die so gewählt sind, daß die Summe (n + m) im Bereich von 1 bis 1000 liegt,
- n ist eine Zahl im Bereich von 0 bis 999 und m ist eine Zahl im Bereich von 1 bis 1000,
- R1, R2 und R3, die gleich oder verschieden sind, bedeuten eine Hydroxygruppe oder eine C1-4-Alkoxygruppe,
- wobei mindestens eine der Gruppen R1 bis R3 eine Hydroxygruppe bedeutet;

Weitere erfindungsgemäß bevorzugte Verfahren sind gekennzeichnet durch die vorangehende Anwendung eines Färbemittels auf dem keratinischem Material, wobei das Färbemittel mindestens aminofunktionelles Silikonpolymer der Formel der Formel (Si-IV) enthält,

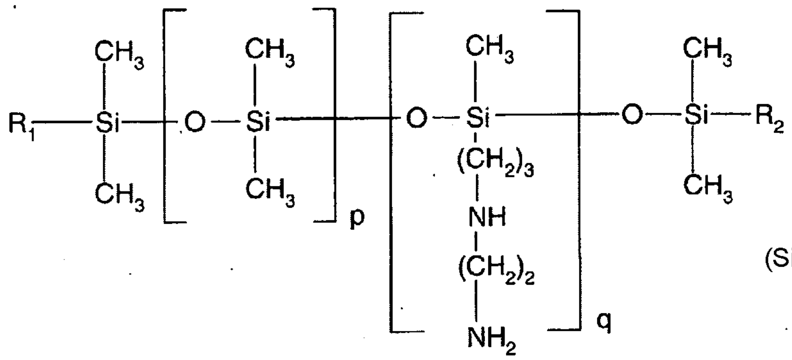

(Si-IV)

in der

- p und q bedeuten Zahlen, die so gewählt sind, daß die Summe (p + q) im Bereich von 1 bis 1000 liegt,
- p ist eine Zahl im Bereich von 0 bis 999 und q ist eine Zahl im Bereich von 1 bis 1000,
- R1 und R2, die verschieden sind, bedeuten eine Hydroxygruppe oder eine C1-4-Alkoxygruppe, wobei mindestens eine der Gruppen R1 bis R2 eine Hydroxygruppe bedeutet.

Die Silikone der Formeln (Si-III) und (Si-IV) unterscheiden sich durch die Gruppierung am Si-Atom, das die stickstoffhaltige Gruppe trägt: In Formel (Si-III) bedeutet R2 eine Hydroxygruppe oder eine C1-4-Alkoxygruppe, während der Rest in Formel (Si-IV) eine Methylgruppe ist. Die einzelnen Si-Gruppierungen, die mit den Indices m und n bzw. p und q gekennzeichnet sind, müssen nicht als Blöcke vorliegen, vielmehr können die einzelnen Einheiten auch statistisch verteilt vorliegen, d.h. in den Formeln (Si-III) und (Si-IV) ist nicht zwingend jedes R1-$Si(CH_3)_2$-Gruppe an eine -[O-$Si(CH_3)_2$]-Gruppierung gebunden.

Als besonders wirkungsvoll im Hinblick auf die Erzeugung von intensiven Farbresultaten haben sich auch erfindungsgemäßen Verfahren erwiesen, in welchen ein Färbemittel auf den Keratinfasern appliziert wird, welches mindestens ein aminofunktionelles Silikonpolymer der Formel der Formel (Si-V) enthält

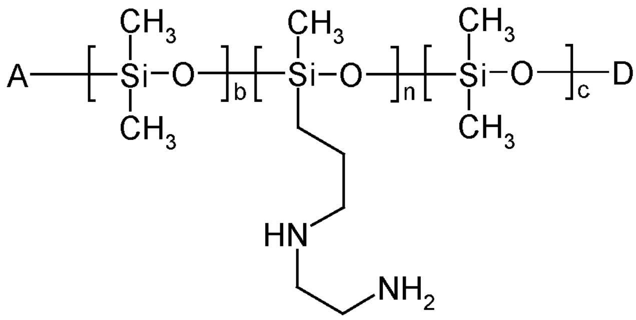

(Si-V),

in der

A für eine Gruppe -OH, -O-Si$(CH_3)_3$,-O-Si$(CH_3)_2$OH ,-O-Si$(CH_3)_2OCH_3$ steht,
D für eine Gruppe -H, -Si$(CH_3)_3$,-Si$(CH_3)_2$OH, -Si$(CH_3)_2OCH_3$ steht,
b, n und c für ganze Zahlen zwischen 0 und 1000 stehen,
mit den Maßgaben

- $n > 0$ und $b + c > 0$
- mindestens eine der Bedingungen A = -OH bzw. D = -H ist erfüllt.

In der vorstehend genannten Formel (Si-V) sind die einzelnen Siloxaneinheiten mit den Indices b, c und n statistisch verteilt, d.h. es muß sich nicht zwingend um Blockcopolymere handeln.

Das zuvor applizierte Färbemittel kann weiterhin auch ein oder mehrere verschiedene aminofunktionalisierte Silikonpolymere enthalten, die durch die Formel (Si-VI)

$$M(R_aQ_bSiO_{(4-a-b)/2)x}(R_cSiO_{(4-c)/2)y}M \qquad \text{(Si-VI)}$$

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel $-R^1HZ$ ist, worin $R^1$ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stand der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von $R^1$ schließen Methylen, Ethylen, Propylen, Hexamethylen, Decamethylen, -$CH_2CH(CH_3)CH_2$-, Phenylen, Naphthylen, -$CH_2CH_2SCH_2CH_2$-, -$CH_2CH_2OCH_2$-, -$OCH_2CH_2$-, -$OCH_2$ $CH_2CH_2$-, -$CH_2CH(CH_3)C(O)OCH_2$-, -$(CH_2)_3$ $CC(O)OCH_2CH_2$-, -$C_6H$ $_4C_6H_4$-, -$C_6H$ $_4CH_2C_6H_4$-; und -$(CH_2)_3C(O)SCH_2CH_2$- ein.

Z ist ein organischer, aminofunktioneller Rest, enthaltend mindestens eine funktionelle Aminogruppe. Eine mögliche Formel für Z ist $NH(CH_2)_zNH_2$, worin z 1 oder mehr ist. Eine andere mögliche Formel für Z ist -$NH(CH_2)_z(CH_2)_{zz}NH$, worin sowohl z als auch zz unabhängig 1 oder mehr sind, wobei diese Struktur Diamino-Ringstrukturen umfaßt, wie Piperazinyl. Z ist am bevorzugtesten ein -$NHCH_2CH_2NH_2$-Rest. Eine andere mögliche Formel für Z ist - $N(CH_2)_z(CH_2)_{zz}NX_2$ oder -$NX_2$, worin jedes X von $X_2$ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel -$CH_2CH_2CH_2NHCH_2CH_2NH$ $_2$. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der $R_aQ_b$ $SiO_{(4-a-b)/2}$-Einheiten zu den $R_cSiO$ $_{(4-c)/2}$-Einheiten liegt im Bereich von etwa 1 : 2 bis 1 : 65, vorzugsweise von etwa 1 : 5 bis etwa

1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Im Rahmen einer besonders bevorzugten Ausführungsform ist ein erfindungsgemäßes Verfahren gekennzeichnet durch die vorangehende Anwendung eines Färbemittels auf dem keratinischem Material, wobei das Färbemittel ein aminofunktionelles Silikonpolymer der Formel (Si-VII)

$$R'_aG_{3-a-}Si(OSiG2)n-(OSiG_bR'_{2-b})_m-O-SiG_{3-a}-R'_a \qquad (Si\text{-}VII),$$

enthält, worin bedeutet:

- G ist-H, eine Phenylgruppe, -OH, $-O-CH_3$, $-CH_3$, $-O-CH_2CH_3$, $-CH_2CH_3$, $-O-CH_2CH_2CH_3$,$-CH_2CH_2CH_3$, $-O-CH(CH_3)_2$, $-CH(CH_3)_2$, $-O-CH_2CH_2CH_2CH_3$, - $CH_2CH_2CH_2CH_3$, $-O-CH_2CH(CH_3)_2$, $-CH_2CH(CH_3)_2$, $-O-CH(CH_3)CH_2CH_3$, - $CH(CH_3)CH_2CH_3$, $-O-C(CH_3)_3$, $-C(CH_3)_3$ ;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
    - $-Q-N(R'')-CH_2-CH_2-N(R'')_2$
    - $-Q-N(R'')_2$
    - $-Q-N^+(R'')_3A^-$
    - $-Q-N^+H(R'')_2\ A^-$
    - $-Q-N^+H_2(R'')A^-$
    - $-Q-N(R'')-CH_2-CH_2-N^+R''H_2A^-$,

  wobei jedes Q für eine chemische Bindung, $-CH_2-$, $-CH_2-CH_2-$, $-CH_2CH_2CH_2-$, $-C(CH_3)_2-$ , $-CH_2CH_2CH_2CH_2-$, $-CH_2C(CH_3)_2-$, $-CH(CH_3)CH_2CH_2-$ steht,
  R'' für gleiche oder verschiedene Reste aus der Gruppe -H, -Phenyl, -Benzyl, $-CH_2-CH(CH_3)Ph$, der $C_{1-20}$-Alkylreste, vorzugsweise $-CH_3$, $-CH_2CH_3$, $-CH_2CH_2CH_3$, - $CH(CH_3)_2$, $-CH_2CH_2CH_2H_3$, $-CH_2CH(CH_3)_2$, $-CH(CH_3)CH_2CH_3$, $-C(CH_3)_3$, steht und A ein Anion repräsentiert, welches vorzugsweise ausgewählt ist aus Chlorid, Bromid, Iodid oder Methosulfat.

Im Rahmen einer weiteren bevorzugten Ausführungsform ist ein erfindungsgemäßes Verfahren gekennzeichnet durch die vorangehende Anwendung eines Färbemittels auf dem keratinischem Material, wobei das Färbemittel mindestens ein aminofunktionelles Silikonpolymer der Formel (Si-VIIa) enthält,

$(CH_3)_3Si-[O-Si(CH_3)_2]_n[OSi(CH_3)]_m-OSi(CH_3)_3$ (Si-VIIa),
|
$CH_2CH(CH_3)CH_2NH(CH_2)_2NH_2$

worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Im Rahmen einer weiteren bevorzugten Ausführungsform ist ein erfindungsgemäßes Verfahren gekennzeichnet durch die vorangehende Anwendung eines Färbemittels auf dem keratinischem Material, wobei das Färbemittel mindestens ein aminofunktionelles Silikonpolymer der Formel (Si-VIIb) enthält

$R-[Si(CH_3)_2-O]_{n1}[Si(R)-O]_m-[Si(CH_3)_2]_{n2}-R$ (Si-VIIb),
|
$(CH_2)_3NH(CH_2)_2NH_2$

enthalten, worin R für -OH, $-O-CH_3$ oder eine $-CH_3$-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese aminofunktionalisierten Siliconpolymere werden nach der INCI-Deklaration als Amodimethicone bezeichnet.

Unabhängig davon, welche aminofunktionellen Silicone eingesetzt werden, sind erfindungsgemäße Färbemittel bevorzugt, die ein aminofunktionelles Silikonpolymer enthalten, dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Weiterhin sind auch Färbemittel zum Einsatz im erfindungsgemäßen Verfahren geeignet, welche ein spezielles 4-Morpholinomethyl-substituiertes Silikonpolymer enthielten. Dieses aminofunktionalisierte Silikonpolymer umfasst Struktureinheiten der Formeln (SI-VIII) und der Formel (Si-IX)

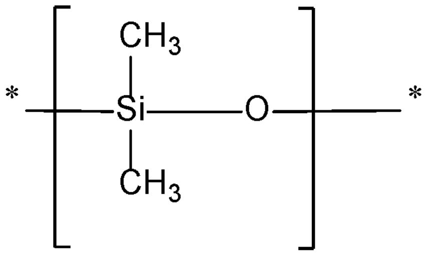

(Si-VIII)

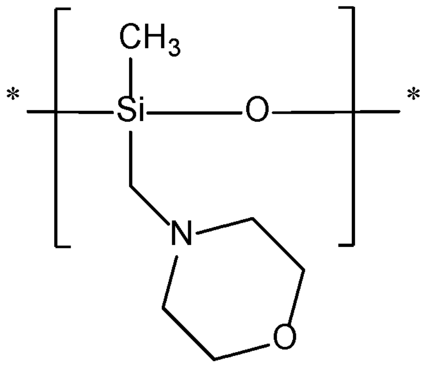

(Si-IX).

Entsprechende 4-Morpholinomethyl-substituiertes Silikonpolymere werden im folgenden beschrieben.

Ein entsprechendes aminofunktionalisertes Silikonpolymer ist unter dem Namen Amodimethicone/Morpholinomethyl Silsesquioxane Copolymer bekannt und in Form des Rohstoffes Belsil ADM 8301 E von Wacker kommerziell erhältlich.

Als 4-morpholinomethyl-substituiertes Silikon kann beispielsweise ein Silikon eingesetzt werden, welches Struktureinheiten der Formeln (Si-VIII), (Si-IX) und (Si-X) aufweist

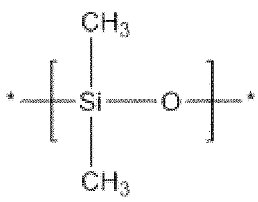

(Si-VIII),

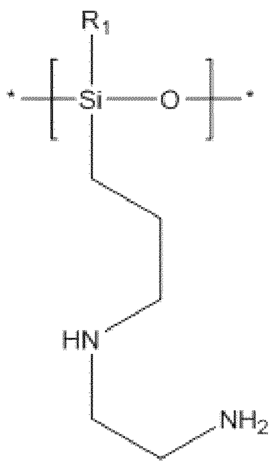

(Si-X)

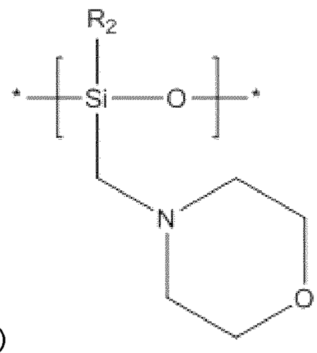

(Si-IX)

in denen

R1 für $-CH_3$, -OH, $-OCH_3$, $-O-CH_2CH_3$, $-O-CH_2CH_2CH_3$, oder $-O-CH(CH_3)_2$ steht;
R2 für $-CH_3$, -OH, oder $-OCH_3$ steht.

Besonders bevorzugte Färbemittel enthalten mindestens ein 4-morpholinomethyl-substituierten Silikons der Formel (Si-XI)

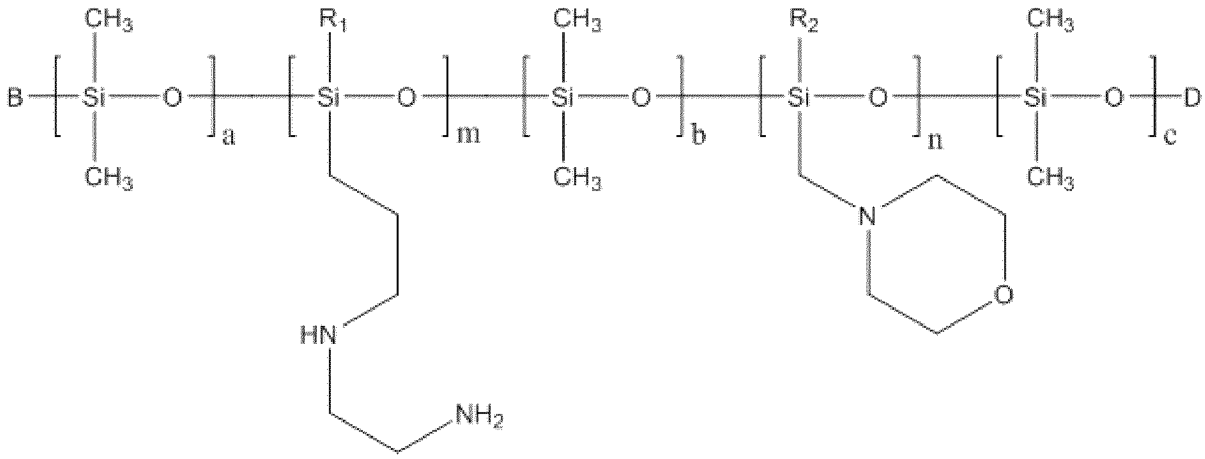

(Si-XI)

in der

R1 für $-CH_3$, -OH, $-OCH_3$, $-O-CH_2CH_3$, $-O-CH_2CH_2CH_3$, oder $-O-CH(CH_3)_2$ steht;
R2 für $-CH_3$, -OH, oder $-OCH_3$ steht.
B für eine Gruppe -OH, $-O-Si(CH_3)_3$,$-O-Si(CH_3)_2OH$ ,$-O-Si(CH_3)_2OCH_3$ steht,
D für eine Gruppe -H, $-Si(CH_3)_3$,$-Si(CH_3)_2OH$, $-Si(CH_3)_2OCH_3$ steht,
a, b und c unabhängig voneinander für ganze Zahlen zwischen 0 und 1000 stehen, mit der Maßgabe a + b + c > 0
m und n unabhängig voneinander für ganze, Zahlen zwischen 1 und 1000 stehen
mit den Maßgabe, daß

- mindestens eine der Bedingungen B = -OH bzw. D = -H erfüllt ist,
- die Einheiten a, b, c, m und n statistisch oder blockweise im Molekül verteilt vorliegen.

Strukturformel (Si-XI) soll verdeutlichen, daß die Siloxangruppen n und m nicht zwingend direkt an eine Endgruppierung B bzw. D gebunden sein müssen. Vielmehr gilt in bevorzugten Formeln (Si-VI) a > 0 oder b > 0 und in besonders bevorzugten Formeln (Si-VI) a > 0 und c > 0, d.h. die terminale Gruppierung B bzw. D ist vorzugsweise an eine Dimethylsiloxy-Gruppierung gebunden. Auch in Formel (Si-VI) sind die Siloxaneinheiten a, b, c, m und n vorzugsweise statistisch verteilt.

Die durch Formel (Si-VI) dargestellten erfindungsgemäß eingesetzten Silikone können trimethylsilylterminiert sein (D oder B = $-Si(CH_3)_3$), sie können aber auch zweiseitig dimethylsilylhydroxy- oder einseitig dimethylsilylhydroxy- und dimethylsilylmethoxy-terminiert sein. Im Rahmen der vorliegenden Erfindung besonders bevorzugt eingesetzte Silikone sind ausgewählt aus Silikonen, in denen

| | | |
|---|---|---|
| B = $-O-Si(CH_3)_2OH$ | und | D = $-Si(CH_3)_3$ |
| B = $-O-Si(CH_3)_2OH$ | und | D = $-Si(CH_3)_2OH$ |
| B = $-O-Si(CH_3)_2OH$ | und | D = $-Si(CH_3)_2OCH_3$ |
| B = $-O-Si(CH_3)_3$ | und | D = $-Si(CH_3)_2OH$ |
| B = $-O-Si(CH_3)_2OCH_3$ | und | D = $-Si(CH_3)_2OH$ |

bedeutet. Diese Silikone führen zu exorbitanten Verbesserungen der Haareigenschaften der mit den erfindungsgemäßen Mitteln behandelten Haare, und zu einem gravierend verbesserten Schutz bei oxidativer Behandlung.

Bei dem im erfindungsgemäßen Verfahren zur vorangehenden Färbung eingesetzten Mittel können ein oder mehrere aminofunktionalisierte Silikonpolymeren beispielsweise in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthalten. Hierbei sind die Mengenangaben auf die Gesamtmenge aller eingesetzten Aminosilikone bezogen, die zum Gesamtgewicht des Färbemittels in Relation gesetzt wird.

Im Rahmen einer weiteren besonders bevorzugten Ausführungsform ist ein erfindungsgemäßes Verfahren dadurch gekennzeichnet, dass das Färbemittel - bezogen auf das Gesamtgewicht des Mittels - ein oder mehrere aminofunktionalisierte Silikonpolymere in einer Gesamtmenge von 0,1 bis 8,0 Gew.-%, bevorzugt 0,2 bis 5,0 Gew.-%,

weiter bevorzugt von 0,3 bis 3,0 Gew.-% und ganz besonders bevorzugt von 0,4 bis 2,5 Gew.-% enthält.

Pigmente im Färbemittel

Im erfindungsgemäßen Verfahren wird ein Entfärbemittel auf Keratinmaterial appliziert, das zuvor durch Anwendung mindestens eines Pigments gefärbt wurde.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente können anorganischen und/oder organischen Ursprungs sein. In einer bevorzugten Ausführungsform ist ein erfindungsgemäßes Verfahren dadurch gekennzeichnet, dass dass das Entfärbemittel auf Keratinmaterial aufgetragen wird, welches durch Anwendung von mindestens einem anorganischen und/oder organischen Pigment gefärbt wurde.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte Farbpigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer bevorzugten Ausführungsform ist ein erfindungsgemäßes Verfahren dadurch gekennzeichnet, dass das Entfärbemittel auf Keratinmaterial aufgetragen wird, welches durch Anwendung von mindestens einem anorganischen Pigment gefärbt wurde, wobei das anorganische Pigment bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

In einer bevorzugten Ausführungsform ist ein erfindungsgemäßes Verfahren dadurch gekennzeichnet, dass das Entfärbemittel auf Keratinmaterial aufgetragen wird, welches durch Anwendung von mindestens einem Pigment gefärbt wurde, das ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Beispiele für besonders geeignete Farbpigmente sind im Handel beispielsweise unter den Handelsbezeichnungen Rona®, Colorona®, Xirona®, Dichrona® und Timiron® von der Firma Merck, Ariabel® und Unipure® von der Firma Sensient, Prestige® von der Firma Eckart Cosmetic Colors und Sunshine® von der Firma Sunstar erhältlich.

Ganz besonders bevorzugte Farbpigmente mit der Handelsbezeichnung Colorona® sind beispielsweise:

Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491(Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491(Iron oxides)
Colorona Blackstar Gold, Merck, MICA, CI 77499 (IRON OXIDES)

Weiterhin besonders bevorzugte Farbpigmente mit der Handelsbezeichnung Xirona® sind beispielsweise:

Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.

Zudem sind besonders bevorzugte Farbpigmente mit der Handelsbezeichnung Unipure® beispielsweise:

Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Im Rahmen einer weiteren Ausführungsform kann das zuvor applizierte Färbemittel auch ein oder mehrere organischen Pigmente enthalten.

Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI

15880, CI 17200, CI 26100, CI 45380, CI 45410, CI 58000, CI 73360, CI 73915 und/oder CI 75470 genannt werden.

In einer weiteren besonders bevorzugten Ausführungsform ist ein erfindungsgemäßes Verfahren, dadurch gekennzeichnet, dass das Entfärbemittel auf Keratinmaterial aufgetragen wird, welches durch Anwendung von mindestens einem organischen Pigment gefärbt wurde, wobei das organische Pigment bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Aufgrund ihrer ausgezeichneten Licht- und Temperaturbeständigkeit ist die Verwendung der zuvor genannten Pigmente in dem Mittel ganz besonders bevorzugt. Weiterhin ist es bevorzugt, wenn die eingesetzten Pigmente eine bestimmte Teilchengröße aufweisen. Es ist daher erfindungsgemäß vorteilhaft, wenn das mindestens eine Pigment eine mittlere Teilchengröße $D_{50}$ von 1,0 bis 50 $\mu$m, vorzugsweise von 5,0 bis 45 $\mu$m, bevorzugt von 10 bis 40 $\mu$m, insbesondere von 14 bis 30 $\mu$m, aufweist. Die mittlere Teilchengröße $D_{50}$ kann beispielsweise unter Verwendung von dynamischer Lichtstreuung (DLS) bestimmt werden.

Bei dem im erfindungsgemäßen Verfahren zur vorangehenden Färbung eingesetzten Mittel können ein oder mehrere Pigmente beispielsweise in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthalten. Hierbei sind die Mengenangaben auf die Gesamtmenge aller eingesetzten Pigmente bezogen, die zum Gesamtgewicht des Färbemittels in Relation gesetzt wird.

In einer weiteren ganz besonders bevorzugten Ausführungsform ist ein erfindungsgemäßes Färbemittel dadurch gekennzeichnet, dass das Färbemittel - bezogen auf das Gesamtgewicht des Färbemittels - ein oder mehrere Pigmente in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

Als weiteren optionalen Bestanteil könnten die Färbemittel zusätzlich auch noch auch einen oder mehrere direktziehende Farbstoffe enthalten. Bei direktziehenden Farbstoffen handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Die direktziehenden Farbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die direktziehenden Farbstoffe im Sinne der vorliegenden Erfindung eine Löslichkeit in Wasser (760 mmHg) bei 25 °C von mehr als 1,0 g/L.

Direktziehende Farbstoffe können in anionische, kationische und nichtionische direktziehende Farbstoffe unterteilt werden.

In einer weiteren Ausführungsform kann ein erfindungsgemäßes Mittel dadurch gekennzeichnet sein, dass es zusätzlich mindestens eine farbgebende Verbindung aus der Gruppe der anionischen, nichtionischen und kationischen direktziehenden Farbstoffe enthält.

Geeignete kationische direktziehende Farbstoffe sind beispielsweise Basic Blue 7, Basic Blue 26, HC Blue 16, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Basic Yellow 57, Basic Yellow 87, Basic Orange 31, Basic Red 51 Basic Red 76.

Als nichtionische direktziehende Farbstoffe können beispielsweise nichtionische Nitro- und Chinonfarbstoffe und neutrale Azofarbstoffe eingesetzt werden. Geeigente nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Anionische direktziehende Farbstoffe werden auch als Säurefarbstoffe bezeichnet. Unter Säurefarbstoffen werden direktziehende Farbstoffe verstanden, die mindestens eine Carbonsäuregruppierung (-COOH) und/oder eine Sulfonsäuregruppierung (-$SO_3H$) besitzen. In Abhängigkeit vom pH-Wert liegen die protonierten Formen (-COOH, -$SO_3H$) der Carbonsäure- bzw. Sulfonsäuregruppierungen mit ihren deprotonierten Formen (-$COO^-$, -$SO_3^-$ vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalente an Kationen neutralisiert. Erfindungsgemäße Säurefarbstoffe können auch in Form ihrer Natriumsalze und/oder ihrer Kaliumsalze eingesetzt werden.

Die Säurefarbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die Säurefarbstoffe im Sinne der vorliegenden Erfindung besitzen eine Löslichkeit in Wasser (760 mmHg) bei 25 °C von mehr als 1,0 g/L.

Die Erdalkalisalze (wie beispielsweise Calciumsalze und Magnesiumsalze) bzw. Aluminiumsalze von Säurefarbstoffen besitzen oftmals eine schlechtere Löslichkeit als die entsprechenden Alkalisalze. Sofern die Löslichkeit dieser Salze unterhalb von 0,5 g/L (25 °C, 760 mmHg) liegt, fallen diese nicht unter die Definition eines direktziehenden Farbstoffes.

Ein wesentliches Merkmal der Säurefarbstoffe ist ihr Vermögen, anionische Ladungen auszubilden, wobei die hierfür verantwortlichen Carbonsäure- bzw. Sulfonsäuregruppen üblicherweise an verschiedene chromophore Systeme geknüpft sind. Geeignete chromophore Systeme finden sich beispielsweise in den Strukturen von Nitrophenylendiaminen, Nitroaminophenolen, Azofarbstoffen, Anthrachinonfarbstoffen, Triarylmethanfarbstoffen, Xanthen-Farbstoffen, Rhodamin-Farbstoffen, Oxazinfarbstoffen und/oder Indophenolfarbstoffen.

Im Rahmen einer weiteren Ausführungsform kann ein Mittel zum Färben von keratinischem Material dadurch gekennzeichnet sein, dass es mindestens einen anionischen direktziehenden Farbstoff enthält, der ausgewählt ist aus der Gruppe der Nitrophenylendiamine, der Nitroaminophenole, der Azofarbstoffe, der Anthrachinonfarbstoffe, der Triarylmethanfarbstoffe, der Xanthen-Farbstoffe, der Rhodamin-Farbstoffe, der Oxazinfarbstoffen und/oder der Indophenolfarbstoffe, wobei die Farbstoffe aus der vorgenannten Gruppe jeweils mindestens eine Carbonsäuregruppe (-COOH), eine Natriumcarboxylatgruppe (-COONa), eine Kaliumcarboxylatgruppe (-COOK), eine Sulfonsäuregruppe (-$SO_3H$) eine Natriumsulfonatgruppe (-$SO_3Na$) und/oder eine Kaliumsulfonatgruppe (-$SO_3K$) besitzen.

Als geeignete Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403,CI 10316, COLIPA n° B001), Acid Yellow 3 (COLIPA n° : C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W 1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201;nosodiumsalt;Brown No.201;RESORCIN BROWN;ACID ORANGE 24;Japan Brown 201;D & C Brown No.1), Acid Red 14 (C.1.14720), Acid Red 18 (E124, Red 18; CI 16255), Acid Red 27 (E 123, CI 16185, C-Rot 46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, Iodeosin), Acid Red 52 (CI 45100, Food Red 106, Solar Rhodamine B, Acid Rhodamine B, Red n° 106 Pontacyl Brilliant Pink), Acid Red 73 (CI CI 27290), Acid Red 87 (Eosin, CI 45380), Acid Red 92 (COLIPA n° C53, CI 45410), Acid Red 95 (CI 45425, Erythtosine,Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.1.42100), Acid Green 22 (C.1.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Die Wasserlöslichkeit der anionischen direktziehenden Farbstoffe kann beispielsweise auf dem folgenden Weg bestimmt werden. 0,1 g des anionischen direktziehenden Farbstoffes werden in ein Becherglas gegeben. Es wird ein Rührfisch hinzugegeben. Dann werden 100 ml Wasser hinzugefügt. Diese Mischung wird auf einem Magnetrührer unter Rühren auf 25 °C erwärmt. Es wird für 60 Minuten gerührt. Danach wird die wässrige Mischung visuell beurteilt. Sind noch

ungelöste Reste vorhanden, wird die Wassermenge - beispielsweise in Schritten von 10 ml - erhöht. Es wird solange Wasser zugegeben, bis sich die eingesetzte Farbstoffmenge komplett gelöst hat. Sofern sich die Farbstoff-Wasser-Mischung aufgrund der hohen Intenstität des Farbstoffes nicht visuell beurteilten lässt, wird die Mischung filtriert. Bleibt auf dem Filterpapier ein Anteil an ungelösten Farbstoffen zurück, wird der Löslichkeitsversuch mit einer höheren Wassermenge wiederholt. Lösen sich 0,1 g des anionischen direktziehenden Farbstoffes bei 25 °C in 100 ml Wasser, so liegt die Löslichkeit des Farbstoffes bei 1,0 g/L.

Acid Yellow 1 trägt den Namen 8-Hydroxy-5,7-dinitro-2-naphthalenesulfonsäure Dinatriumsalz und besitzt eine Löslichkeit in Wasser von mindestens 40 g/L (25°C).

Acid Yellow 3 ist ein Gemisch der Natriuimsalze von Mono- und Sisulfonsäuren von 2-(2-Chinolyl)-1H-indene-1,3(2H)-dion und besitzt eine Wasserlöslichkeit von 20 g/L (25 °C).

Acid Yellow 9 ist das Dinatriumsalz der 8-Hydroxy-5,7-dinitro-2-naphthalenesulfonsäure, seine Wasserlöslichkeit liegt oberhalb von 40 g/L (25 °C).

Acid Yellow 23 ist das Trinatriumsalz der4,5-Dihydro-5-oxo-1-(4-sulfophenyl)-4-((4-sulfophenyl)azo)-1H-pyrazole-3-carbonsäure und bei 25 °C gut in Wasser löslich.

Acid Orange 7 ist das Natriumsalz des 4-[(2-Hydroxy-1-naphthyl)azo]benzensulfonats. Seine Wasserlöslichkeit beträgt mehr als 7 g/L (25 °C).

Acid Red 18 ist das Trinatirumsalz von 7-Hydroxy-8-[(E)-(4-sulfonato-1-naphthyl)-diazenyl)]-1,3-naphthalenedisulfonat und besitzt eine sehr hohe Wasserlöslichkeit von mehr als 20 GEw.-%.

Acid Red 33 ist das Diantriumsalz des 5-Amino-4-hydroxy-3-(phenylazo)-naphthalene-2,7-disulphonats, seine Wasserlöslichkeit liegt bei 2,5 g/L (25 °C).

Bei Acid Red 92 handelt es sich um das Dinatriumsalz der 3,4,5,6-Tetrachloro-2-(1 ,4,5,8-tetrabromo-6-hydroxy-3-oxoxanthen-9-yl)benzoesäure, dessen Wasserlöslichkeit mit größer 10 g/L angegeben wird (25 °C).

Acid Blue 9 ist das Dinatriumsalz von 2-({4-[N-ethyl(3-sulfonatobenzyl]amino]phenyl}{4-[(N-ethyl(3-sulfonatobenzyl)imino]-2,5-cyclohexadien-1-ylidene}methyl)-benzenesulfonat und besitzt eine Wasserlöslichkeit von mehr als 20 Gew.-% (25 °C).

In einer weiteren Ausführungsform ist ein erfindungsgemäßes Färbemittel daher dadurch gekennzeichnet, dass es mindestens einen direktziehenden Farbstoff enthält, der ausgewählt ist aus der Gruppe aus Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red, Acid Red 27, Acid Red 33, Acid Red 35, Acid Red 51, Acid Red 52, Acid Red 73, Acid Red 87, Acid Red 92, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Der oder die direktziehenden Farbstoffe können je nach erwünschter Farbintensität in verschiedenen Mengen im Färbemittel eingesetzt werden. Gute Ergebnisse konnten erhalten werden, wenn das Färbemittel - bezogen auf das Gesamtgewicht des Färbemittel - ein oder mehrere direktziehende Farbstoffe in einer Gesamtmenge von 0,01 bis 10,0 Gew.-%, bevorzugt von 0,1 bis 8,0 Gew.-%, weiter bevorzugt von 0,2 bis 6,0 Gew.-% und ganz besonders bevorzugt von 0,5 bis 4,5 Gew.-% enthält.

Weiterhin kann das Mittel als zusätzlichen optionalen Bestandteil auch noch eine farbgebende Verbindung aus der Gruppe der photochromen oder thermochromen Farbstoff enthalten.

Photochrome Farbstoffe sind Farbstoffe, die auf Bestrahlung mit UV-Licht (Sonnenlicht oder Schwarzlicht) mit einer reversiblen Farbtonänderung reagieren. Dabei verändert das UV-Licht die chemische Struktur der Farbstoffe und damit ihr Absorptionsverhalten (Photochromie).

Thermochrome Farbstoffe sind Farbstoffe, die auf Temperaturänderungen mit einer reversiblen Farbtonänderung reagieren. Dabei verändert die Temperaturänderung die chemische Struktur der Farbstoffe und damit ihr Absorptionsverhalten (Thermochromie).

Das Färbemittel kann - bezogen auf das Gesamtgewicht des Färbemittels - ein oder mehrere photochrome und/oder thermochrome Farbstoffe in einer Gesamtmenge von 0,01 bis 10,0 Gew.-%, bevorzugt von 0,1 bis 8,0 Gew.-%, weiter bevorzugt von 0,2 bis 6,0 Gew.-% und ganz besonders bevorzugt von 0,5 bis 4,5 Gew.-% enthalten.

<u>Entfärbemittel</u>

Im Rahmen des erfindungsgemäßen Verfahrens wird auf das Keratinmaterial, insbesondere auf menschliche Haare, das bzw. die wie zuvor beschrieben gefärbt wurden, ein Entfärbemittel appliziert. Das Entfärbemittel wird auf das gefärbte Keratinmaterial aufgetragen und nach einer Einwirkzeit wieder abgespült.

Der Zeitpunkt, zu dem das Entfärbemittel angewendet wird, hängt von den Bedürfnissen das Anwenders ab und kann an dessen Gewohnheiten angepasst werden.

Beispielsweise ist es möglich, dass Entfärbemittel auf das frisch gefärbte, noch nasse oder bevorzugt getrocknete Keratinmaterial aufzutragen, so dass zwischen dem Ausspülen des Färbemittels und der Anwendung des Entfärbemittels ein Zeitraum von einigen Stunden liegt. Diese Anwendung des Entfärbemittels kurz nach der Färbung kann insbesondere dann vorgenommen werden, wenn das Ergebnis der Färbung nicht mit den Erwartungen des Anwenders übereinstimmt. Auch ist es beispielsweise möglich, eine sehr intensive oder auffällige Färbung zu einem bestimmten Anlass vorzunehmen und die Färbung nach diesem Anlass wieder zu entfernen.

Ebenso ist es möglich, dass zwischen der vorangehenden Anwendung des Färbemittels und der Anwendung des Entfärbemittels ein längerer Zeitraum liegt, der bei einigen bis mehreren Tagen oder sogar Wochen liegen kann. In diesem Zusammenhang gilt die Voraussetzung, dass das Entfärbemittel auf gefärbtes Keratinmateral aufgetragen wird, was bedeutet, dass das Keratinmaterial immer noch durch die Anwendung der Pigmente gefärbt sein muss.

Das Entfärbemittel ist dadurch gekennzeichnet, dass es

(a) mindestens ein Salz eines ein- oder zweiwertigen Kations enthält, und
(b) einen pH-Wert von 1,0 bis 4,7 besitzt.

<u>Salze von ein- oder zweiwertigen Kationen im Entfärbemittel</u>

Das im erfindungsgemäßen Verfahren eingesetzte Entfärbemittel ist dadurch gekennzeichnet, dass es mindestens ein Salz eines ein- oder zweiwertigen Kations enthält.

Unter einwertigen Kationen werden einfach positive geladene Kationen verstanden. Beispiele für einwertige Kationen sind das Natrium-Kation ($Na^+$), das Kaliumkation ($K^+$) und das AmmoniumKation $(NH_4)^+$.

Unter zweiwertigen Kationen werden zweifach positiv geladene Kationen verstanden. Beispiele für zweiwertige Kationen sind $Ca^{2+}$ (das Calium-Kation), $Mg^{2+}$ (das Magnesium-Kation), Ba2+ (das Barium-Kation), $Cu^{2+}$ (das Kupfer-Kation), $Fe^{2+}$ (das Eisen-Kation) und $Zn^{2+}$ (das Zink-Kation).

Besonders gut geeignete Salze von ein- oder zweiwertigen Kationen sind die Salze des Calciums, die Calze des Magnesiums, die Salze des Natriums, die Salze des Kaliums und die Salze des Ammoniums.

In einer weiteren Ausführungsform ist ein erfindungsgemäßes Verfahren dadurch gekennzeichnet, dass das Entfärbemittel mindestens ein Salz eines ein- oder zweiwertigen Kations (a) enthält, das ausgewählt ist aus der Gruppe der Calciumsalze, der Magnesiumsalze, der Natriumsalze, der Kaliumsalze und der Ammoniumsalze.

In den Salzen der zweiwertigen Kationen werden die beiden positiven Ladungen durch die Anwesenheit der entsprechenden Äquivalente an anionisch geladenen Gegenionen neutralisiert. Bei den als Gegenion vorhandenen Anionen kann es sich um anorganische oder organische Gegenionen handeln.

Beispielhaft für organische anionische Gegenionen können die Citrate (die Salze der Zitronensäure), die Salicylate (die Salze der Salicylsäure), die Tartrate (die Salze der Weinsäure), die Lactate (die Salze der Milchsäure), die Malate (die Salze der Äpfelsäure), die Succinate (die Salze der Bernsteinsäure), die Benzoate (die Salze der Benzoesäure), die Fumarate (die Salze der Fumarsäure), die Maleinate (die Salze der Maleinsäure) und die Acetate (die Salze der Essigsäure) genannt werden.

Beispielhaft für anorganische anionische Gegenionen können die Phosphate, die Sulfate, die Sikate, die Hydrogenphosphate, die Carbonate und die Hydrogencarbonate genannt werden.

In einer weiteren ganz besonders bevorzugten Ausführungsform ist ein erfindungsgemäßes Verfahren dadurch gekennzeichnet, dass das Entfärbemittel mindestens ein Salz eines ein- oder zweiwertigen Kations (a) enthält, das ausgewählt ist aus der Gruppe aus

- Calciumcitrat, Magnesiumcitrat, Natriumcitrat, Ammoniumcitrat,
- Calciumsalicylat, Magnesiumsalicylat, Natriumsalicylat, Ammoniumsalicylat,
- Calciumphosphat, Magnesiumphosphat, Natriumphosphat, Ammoniumphosphat,
- Calciumsulfat, Magnesiumsulfat, Natriumsulfat, Ammoniumsulfat,
- Calciumtartrat, Magnesiumtartrat, Natriumtartrat, Ammoniumtartrat,
- Calciumlactat, Magnesiumlactat, Natriumlactat, Ammoniumlactat,
- Calciummalat, Magnesiummalat, Natriummalat, Ammoniummalat,
- Calciumsuccinat, Magnesiumsuccinat, Natriumsuccinat, Ammoniumsuccinat,
- Calciumbenzoat, Magnesiumbenzoat, Natriumbenzoat, Ammoniumbenzoat,
- Calciumfumarat, Magnesiumfumarat, Natriumfumarat, Ammoniumfumarat,
- Calciummaleinat, Magnesiummaleinat, Natriummaleinat, Ammoniummaleinat,
- Calciumacetat, Magnesiumacetat, Natriumacetat, Ammoniumacetat,
- Calciumchlorid, Magnesiumchlorid, Natriumchlorid, Ammoniumchlorid,
- Calciumbromid, Magnesiumbromid, Natriumbromid und/oder Ammoniumbromid.

Calciumcitrat, das Calciumsalz der Citronensäure, ist ein weißer, kristalliner Feststoff, der die CAS-Nummern 813-94-5 (wasserfrei) und 5785-44-4 (Tetrahydrat) trägt.

Unter dem Begriff Magnesiumcitrat werden die Magnesiumsalze der Citronensäure verstanden. Im Einzelnen können dies sein:

- wasserfreies Magnesiumcitrat mit variablem Citrat-Magnesium-Verhältnis $Mg_x(C_eH_5O_7)_y$, CAS-Nummer: 7779-25-1
- tribasisches Magnesiumcitrat (Trimagnesiumdicitrat, $C_{12}H_{10}Mg_3O_{14}$) und seine Hydrate
- dibasisches Magnesiumcitrat (Magnesiumhydrogencitrat, $C_6H_6MgO_7$) und seine Hydrate

Natriumcitrat ist das Natriumsalz der Citronensäure. Es besteht aus drei Natriumionen ($Na^+$) und dem Citration ($C_6H_5O_7^{3-}$) und trägt die CAS-Nummer 68-04-2.

Calciumsalicylat ist das Calciumsalz der Salicylsäure.

Magnesiumsalicylat ist das Magnesiumsalz der Salicylsäure.

Natriumsalicylat ist das Natriumsalz der Salicylsäure. Es wird alternativ auch als 2-Hydroxybenzoesäure Natriumsalz bezeichnet und trägt die CAS-Nummer 54-21-7.

Calciumphosphat besitzt die Summenformel $Ca_3(PO_4)_2$ und trägt die CAS-Nummer 7758-87-4.

Als Magnesiumphosphate werden in der Lebensmitteltechnik zusammenfassend Magnesiumdihydrogenphosphat Magnesiumhydrogenphosphat und Magnesiumphosphat bezeichnet. Sie sind in der Europäischen Union als Lebensmittelzusatzstoffe unter der gemeinsamen Nummer E 343 zugelassen.

Natriumphosphat ist ein Natriumsalz der Phosphorsäure. Es besteht aus Natrium- (3 $Na^+$) und Phosphationen ($PO_4^{3-}$) und besitzt die CAS-Nummern 7601-54-9, 15819-50-8 (Hexahydrat) und 10101-89-0 (Dodecahydrat).

Calciumsulfat besitzt die Summenformel $CaSO_4$ und trägt die CAS-Nummer 7778-18-9 (wasserfrei) bzw. 10034-76-1 (Hemihydrat), 10101-41-4 (Dihydrat) oder 13397-24-5 (Hydrat).

Magnesiumsulfat besitzt die Summenformel MgSO4 und trägt die CAS-Nummer 7487-88-9 (Magnesiumsulfat) bzw. 10034-99-8 ($MgSO_4 \cdot 7\ H_2O$).

Calciumtartrat ist das Calciumsalz der Weinsäure, üblicherweise der L-Weinsäure und besitzt die CAS-Nummern 3164-34-9, 5892-21-7 (Tetrahydrat) und 110720-66-6 (DL).

Natriumtartrat ist ein Natriumsalz der Weinsäure mit der Summenformel $C_4H_4O_6Na_2$ und besitzt die CAS-Nummern 868-18-8 (Anhydrat), 6106-24-7 (Dihydrat), 109175-69-1 (Anhydrat), 22476-07-9 (Dihydrat), 4504-50-1 (meso-Form) und 51307-92-7 (Racemat).

Calciumlactat ist das Calciumsalz der Milchsäure und trägt die CAS-Nummer 814-80-2. Magnesiumlactat ist das Magnesiumsalz der Milchsäure.

Natriumlactat (Natrium lacticum) ist das Natriumsalz der Milchsäure mit der Formel $NaC_3H_5O_3$ und der CAS-Nummer 72-17-3.

Calciummalat ist das Calciumsalz der Äpfelsäure (E 296) und ein Lebensmittelzusatzstoff. Calciummalat besitzt die CAS-Nummer 17482-42-7.

Magnesiummalat ist das Magnesiumsalz der Äpfelsäure mit der CAS-Nummern 869-06-7 und 6150-86-3 (Trihydrat).

Natriummalat ist das Natriumsalz der Äpfelsäure mit den CAS-Nummern 676-46-0 (unspezifizierte Stereochemie), 22798-10-3 (Racemat), 138-09-0 (L-Form) und 207511-06-6 (L-Form-Hydrat).

Calciumsuccinat ist das Calciumsalz der Bernsteinsäure.

Magnesiumsuccinat ist das Magnesiumsalz der Bernsteinsäure.

Natriumsuccinat ist das Natriumsalz der Bernsteinsäure.

Calciumbenzoat ist das Calciumsalz der Benzoesäure.

Magnesiumbenzoat ist das Magnesiumsalz der Benzoesäure.

Natriumbenzoat ist das Natriumsalz der Benzoesäure.

Calciumfumarat ist das Calciumsalz der Fumarsäure und trägt die CAS-Nummern 7718-51-6 und 19855-56-2.

Magnesiumfumarat ist das Magnesiumsalz der Fumarsäure

Natriumfumarat ist das Natriumsalz der Fumarsäure

Calciumchlorid besitzt die Summenformel $CaCl_2$ und trägt die CAS-Nummern 10043-52-4 (wasserfrei), 13477-29-7 (Monohydrat), 10035-04-8 (Dihydrat), 25094-02-4 (Tetrahydrat), 7774-34-7 (Hexahydrat) und 22691-02-7 (Hydrat).

Calciumbromid besitzt die Summenformel CaBr2 und trägt die CAS-Nummern 7789-41-5 (wasserfrei), 71626-99-8 (Hydrat), 22208-73-7 (Dihydrat) und 13477-28-6 (Hexahydrat). Magnesiumchlorid besitzt die Summenformel MgCl2 und trägt die CAS-Nummern 7786-30-3 (wasserfrei) und 7791-18-6 (Hexahydrat).

Magnesiumbromid besitzt die Summenformel MgBr2 und trägt die CAS-Nummern 7789-48-2 und 13446-53-2 (Hexahydrat).

Für die Optimierung der Entfärbeleistung kann es bevorzugt sein, die Salze der ein- oder zweiwertigen Kationen

(a) in bestimmten Mengenbereichen im Entfärbemittel einzusetzen. Besonders gute Effekte wurden erhalten, wenn das Entfärbemittel - bezogen auf das Gesamtgewicht des Entfärbemittels - ein oder mehrere Salze von ein- oder zweiwertigen Kationen (a) in einer Gesamtmenge von 1,0 bis 15,0 Gew.-%, bevorzugt 1,5 bis 13,0 Gew.-%, weiter bevorzugt 3,0 bis 12,0 Gew.-% und ganz besonders bevorzugt 4,5 bis 8,0 Gew.-% enthielt.

In einer weiteren Ausführungsform ist ein erfindungsgemäßes Verfahren dadurch gekennzeichnet, dass das Entfärbemittel - bezogen auf das Gesamtgewicht des Entfärbemittels - ein oder mehrere Salze von ein- oder zweiwertigen Kationen (a) in einer Gesamtmenge von 1,0 bis 15,0 Gew.-%, bevorzugt 1,5 bis 13,0 Gew.-%, weiter bevorzugt 3,0 bis 12,0 Gew.-% und ganz besonders bevorzugt 4,5 bis 8,0 Gew.-% enthält.

Wassergehalt im Entfärbemittel

Da das Entfärbemittel erfindungsgemäß auf einen sauren pH-Wert im Bereich von 1,0 bis 4,7 eingestellt wird, enthält es Wasser bzw. umfasst einen wasserhaltigen Träger.

Demzufolge ist ein erster Gegenstand der vorliegenden Erfindung mit anderen Worten ein Verfahren zur Entfärbung von Keratinmaterial, welches durch Anwendung mindestens eines Pigments gefärbt wurde, wobei ein Entfärbemittel, welches

(a) mindestens ein amphoteres und/oder zwitterionisches Tensid enthält, und
(b) Wasser enthält und einen pH-Wert von 1,0 bis 4,7 besitzt,

auf das gefärbte Keratinmaterial aufgetragen und nach einer Einwirkzeit wieder abgespült wird.

Eine besonders gute Entfernung der überschüssigen Pigmente bzw. Aminosilikone war möglich, wenn das Entfärbemittel - bezogen auf das Gesamtgewicht des Entfärbemittels -einen Wassergehalt von 50 bis 99 Gew.-%, bevorzgut von 55 bis 98 Gew.-%, weiter bevorzugt von 60 bis 97 Gew.-%, und besonders bevorzugt von 70 bis 96 Gew.-% besaß.

In einer weiteren besonders bevorzugten Ausführungsform ist ein erfindungsgemäßes Verfahren daher dadurch gekennzeichnet, dass das Entfärbemittel - bezogen auf das Gesamtgewicht des Entfärbemittels - einen Wassergehalt von 50 bis 99 Gew.-%, bevorzgut von 55 bis 98 Gew.-%, weiter bevorzugt von 60 bis 97 Gew.-%, und besonders bevorzugt von 70 bis 96 Gew.-% besitzt.

pH-Wert des Entfärbemittels

Kennzeichnend für das Entfärbemittel ist ein saurer pH-Wert im Bereich von 1,0 bis 4,7.

In der zu dieser Erfindung führenden Reihenversuchen hat sich gezeigt, dass durch die Wahl des optimalen pH-Wertes die Entfärbeleistung gesteuert werden konnte. Eine gute Entfärbewirkung konnte bereits ab einem pH-Wert von 4,7 beobachtet werden. Durch ein weiteres Absenken des pH-Wertes konnte diese Entfärbeleistung jedoch noch weiter verbessert werden.

In diesem Zusammenhang hat es sich als ganz besonders bevorzugt herausgestellt, wenn das Entfärbemittel einen pH-Wert (b) von 1,5 bis 4,6, bevorzugt von 2,0 bis 4,6, weiter bevorzugt von 2,5 bis 4,6, noch weiter bevorzugt von 3,0 bis 4,6 und ganz besonders bevorzugt von 3,8 bis 4,6 besitzt.

Im Rahmen einer weiteren ganz besonders bevorzugten Ausführungsform ist ein erfindungsgemäßes Verfahren dadurch gekennzeichnet, dass das Entfärbemittel einen pH-Wert (b) von 1,5 bis 4,6, bevorzugt von 2,0 bis 4,6, weiter bevorzugt von 2,5 bis 4,6, noch weiter bevorzugt von 3,0 bis 4,6 und ganz besonders bevorzugt von 3,8 bis 4,6 besitzt.

Tenside im Entfärbemittel

Das im erfindungsgemäßen Verfahren eingesetzte Entfärbemittel wird ganz besonders bevorzugt in Form eines Shampoo konfektioniert. Es hat sich als ganz besonders vorteilhaft herausgestellt, wenn das Entfärbemittel mindestens ein Tensid, bevorzugt mindestens ein anionisches und/oder zwitterionisches Tensid enthält.

Im Rahmen einer weiteren ganz besonders bevorzugten Ausführungsform ist ein erfindungsgemäßes Verfahren dadurch gekennzeichnet, dass das Entfärbemittel mindestens ein anionisches und/oder zwitterionisches Tensid enthält.

Unter dem Begriff Tenside (T) werden grenzflächenaktive Substanzen, die an Ober- und Grenzflächen Adsorptionsschichten bilden oder in Volumenphasen zu Mizell-Kolloiden oder lyotropen Mesophasen aggregieren können, verstanden. Man unterscheidet anionische Tenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.

Zu den geeigneten anionischen Tensiden, die in den erfindungsgemäßen Entfärbemitteln eingesetzt werden können, zählen:

- lineare und verzweigte Fettsäuren mit 8 bis 30 C-Atomen (Seifen),
- Ethercarbonsäuren der Formel $R\text{-}O\text{-}(CH_2\text{-}CH_2O)_x\text{-}CH_2\text{-}COOH$, in der R eine lineare oder verzweigte, gesättigte oder ungesättigte Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- Alpha-Olefinsulfonate mit 8 bis 24 C-Atomen,
- Alkylsulfat- und/oder Alkylpolyglykolethersulfatsalze der Formel $R\text{-}(OCH_2\text{-}CH_2)_x\text{-}OSO_3^-\ X^+$, in der R eine bevorzugt lineare oder verzweigte, gesättigte oder ungesättigte Alkylgruppe mit 8 bis 30 C-Atomen, x = 0 oder 1 bis 12 und X ein Alkali- oder Ammoniumion ist,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel,

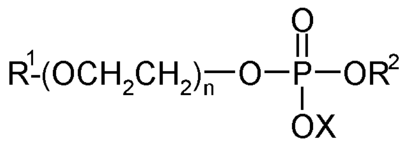

in der $R^1$ bevorzugt für einen aliphatischen Kohlenwasserstoffrest mit 8 bis 30 Kohlenstoffatomen, $R^2$ für Wasserstoff, einen Rest $(CH_2CH_2O)_nR^1$ oder X, n für Zahlen von 0 bis 10 und X für Wasserstoff, ein Alkali- oder Erdalkalimetall oder $NR^3R^4R^5R^6$, mit $R^3$ bis $R^6$ unabhängig voneinander stehend für einen $C_1$ bis $C_4$ -Kohlenwasserstoffrest, steht.

Ganz besonders gute Ergebnisse wurden erhalten, wenn im Entfärbemittel mindestens ein anionisches Tensid der Formel (T-1) eingesetzt wurde,

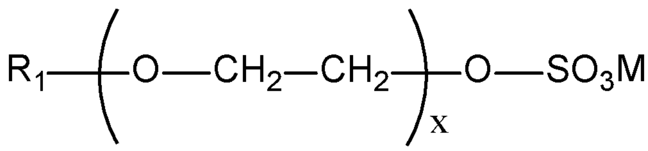

(T-1),

wobei

$R_1$ für eine lineare oder verzweigte, gesättigte oder ungesättigte $C_8$-$C_{30}$-Alkylgruppe steht,
x für eine ganze Zahl von 0 bis 50 steht, und
M für ein Wasserstoffatom, für Ammonium $(NH_4)_+$ oder für ein Äquivalent eines ein oder mehrwertigen Kations steht.

Im Rahmen einer ganz besonders bevorzugten Ausführungsform ist ein erfindungsgemäßes Verfahren dadurch gekennzeichnet, dass das Entfärbemittel mindestens ein anionisches Tensid der Formel (T-1) enthält,

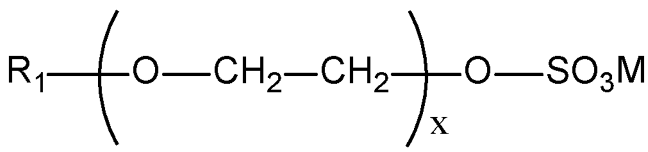

(T-1),

wobei

$R_1$ für eine lineare oder verzweigte, gesättigte oder ungesättigte $C_8$-$C_{30}$-Alkylgruppe steht,
x für eine ganze Zahl von 0 bis 50 steht, und
M für ein Wasserstoffatom, für Ammonium $(NH_4)_+$ oder für ein Äquivalent eines ein oder mehrwertigen Kations steht.

Der Rest R1 stellt den hydrophoben Teil des anionischen Tensids dar und steht für eine lineare oder verzweigte, gesättigte oder ungesättigte $C_8$-$C_{30}$-Alkylgruppe. Wenn der Rest für eine ungesättigte $C_8$-$C_{30}$-Alkylgruppe steht, dann kann die Alkylgruppe einfach oder mehrfach ungesättigt sein.

Bevorzugt steht der Rest R1 für eine lineare, gesättigte oder ungesättigte $C_8$-$C_{30}$-Alkylgruppe- Ganz besonders bevorzugt steht der Rest R1 für eine lineare, gesättigte oder ungesättigte $C_{12}$-$C_{22}$-Alkylgruppe. Explizit ganz besonders bevorzugt steht der Rest R1 für eine R für eine lineare, gesättigte oder ungesättigte $C_{12}$-$C_{18}$-Alkylgruppe.

Beispiele für gesättigte, lineare $C_8$-$C_{30}$-Alkylgruppen sind die Laurylgruppe, die Myristylgruppe, die Cetylgruppe, die Stearylgruppe und die Behenylgruppe.

Die Indexzahl x gibt die Anzahl der im anionischen Tensid enthatlenen Ethylenoxid-Gruppen an. Steht x für die Zahl 0, dann besitzt das anionische Tensid der Formel (T-1) keine EthylenoxidEinheiten, in diesem Fall liegt ein Alkyl-Sulfat bzw. das Salz eines Alkyl-Sulfats vor.

Beispiele für die Salzs von Alkylsulfaten sind Natriumlaurylsulfat und Natriummyristylsulfat.

Bevorzugt steht x für eine ganze Zahl von 0 bis 5, besonders bevorzugt steht x für eine ganze Zahl von 1 bis 5.

Der Rest M steht für ein Wasserstoffatom, für Ammonium $(NH_4)_+$ oder für ein Äquivalent eines ein oder mehrwertigen Kations.

Steht M für ein Wasserstoffatom, dann liegt das anionische Tensid in Form des protonierten (und gegebenenfalls ethoxylierten) Schwefelsäureesters vor. In wässriger Lösung steht die protonierte Form mit der deprotonierten Form im Gleichgewicht, wobei die deprotonierte Form eine anionische Ladung trägt. Aus diesem Grund fallen auch die protonierten Verbindungen der Formel (I) in die Gruppe der anionischen Tenside.

Steht M für Ammonium $(NH_4)_+$ oder für ein Äquivalent eines ein oder mehrwertigen Kations, dann liegt das anionsiche Tensid der Formel (I) in Form seines Salzes vor. Das Vorhandensein der entsprechenden Äquivalent eines ein- oder mehrwertigen Kations gewährleistet hierbei die Elektroneutralität des Anion-Tensid. Bevorzugt steht M für ein einwertiges Kation, insbesondere für ein Natrium- oder Kalium-Kation.

Im Rahmen einer ganz besonders bevorzugten Ausführungsform ist ein erfindungsgemäßes Verfahren dadurch gekennzeichnet, dass das Vorbehandlungsmittel (V) mindestens ein anionisches Tensid (V-1) der Formel (T-1) enthält, wobei

R1 für eine lineare, gesättigte oder ungesättigte $C_{12}$-$C_{18}$-Alkylgruppe steht,
x für eine ganze Zahl von 0 bis 5, bevorzugt für eine ganze Zahl von 1 bis 5, steht,
M für ein Wasserstoffatom oder für ein Alkalimetallkation, bevorzugt für ein Natriumkation oder für ein Kaliumkation, steht.

Ein ganz besonders bevorzugtes anionisches Tensid der Formel (I) kann beispielsweise unter dem Handelsnamen Texapon NSO BZ (BZ = preserved with benzoic acid) und der INCI Bezeichnung Sodium Laureth Sulfate von der Firma BASF kommerziell bezogen werden. Dieses Sodium Laureth Sulfate besitzt die CAS-Nummer 68891-38-3.

Zur Gewährleistung einer besonders guten Entfärbeleistung im erfindungsgemäßen Verfahren enthält das Entfärbemittel - bezogen auf das Gesamtgewicht des Entfärbemittels - ein oder mehrere anionische Tenside in einer Gesamtmenge von 2,0 bis 18,0 Gew.-%, bevorzugt von 4,0 bis 16,0 Gew.-%, weiter bevorzugt von 6,0 bis 14,0 Gew.-% und ganz besonders bevorzugt von 8,0 bis 12,0 Gew.-%.

Im Rahmen einer ganz besonders bevorzugten Ausführungsform ist ein erfindungsgemäßes Verfahren dadurch gekennzeichnet, dass das Entfärbemittel - bezogen auf das Gesamtgewicht des Entfärbemittels - ein oder mehrere anionische Tenside in einer Gesamtmenge von 2,0 bis 18,0 Gew.-%, bevorzugt von 4,0 bis 16,0 Gew.-%, weiter bevorzugt von 6,0 bis 14,0 Gew.-% und ganz besonders bevorzugt von 8,0 bis 12,0 Gew.-% enthält.

Die mit dem erfindungsgemäßen Verfahren erzielbaren Effekte ließen noch weiter verbessern, wenn im Entfärbemittel zusätzlich zum oder anstatt des anionischenTensids mindestens ein zwitterionisches und/oder amphoteres Tensid eingesetzt wurde.

Im Rahmen einer ganz besonders bevorzugten Ausführungsform ist ein erfindungsgemäßes Verfahren dadurch gekennzeichnet, dass das Entfärbemittel mindestens ein zwitterionisches und/oder amphoteres Tensid enthält.

Ganz besonders gut geeignete zwitterionische Tenside (V-2) können ausgewählt sein aus Verbindungen der folgenden Formeln (T-2), (T-3), (T-4) und/oder (T-5),

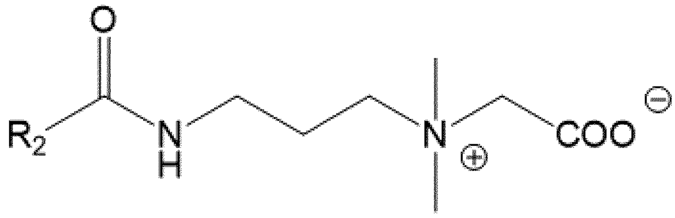

(T-2)

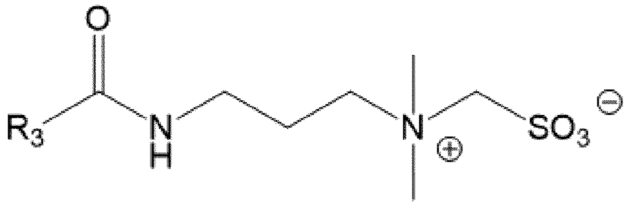

(T-3)

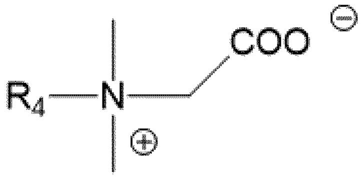

(T-4)

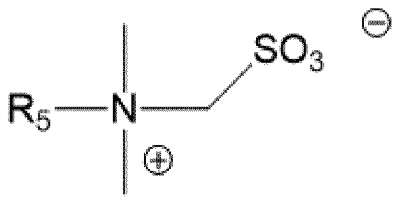

(T-5)

wobei

R2, R3, R4 und R5 jeweils unabhängig voneinander den hydrophoben Rest des Tensids darstellen.
R2, R3, R4 und R5 stehen unabhängig voneinander für eine lineare oder verzweigte, gesättigte oder ungesättigte $C_8$-$C_{30}$-Alkylgruppe, bevorzugt für eine lineare, gesättigte oder ungesättigte $C_{12}$-$C_{18}$-Alkylgruppe.

Die zwitterionischen Tenside der Formel (T-2), (T-3), (T-4) und/oder (T-5) besitzen jeweils eine kationische Ladung in Form eines quartären Stickstoffatoms, welches neben zwei Methylgruppen den jeweiligen Rest R und weiterhin den Rest umfassend die Säurefunktion trägt. Diese kationische Ladung wird durch die Säurefunktion, bei der es sich um eine deprotonierte Carbonsäure oder Sulfonsäure handeln kann, neutralisiert.

Im Rahmen einer ganz besonders bevorzugten Ausführungsform ist ein erfindungsgemäßes Verfahren dadurch gekennzeichnet,dass das Entfärbemittel mindestens ein zwitterionisches Tensid enthält, das ausgewählt ist aus den Tensiden der Formel (T-2), (T-3), (T-4) und/oder (T-5),

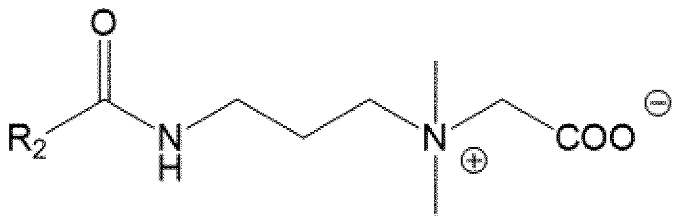

(T-2)

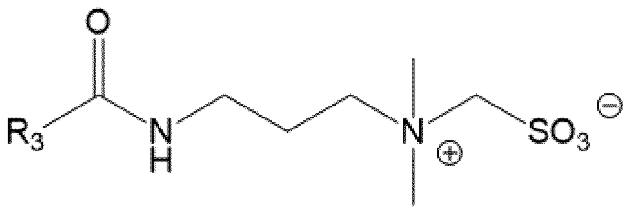

(T-3)

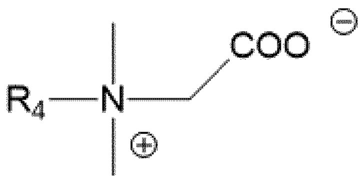

(T-4)

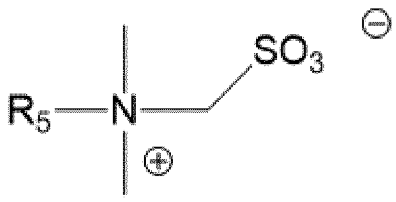

(T-5)

wobei

$R_2$, $R_3$, $R_4$, $R_5$ unabhängig voneinander für eine lineare oder verzweigte, gesättigte oder ungesättigte $C_8$-$C_{30}$-Alkylgruppe, bevorzugt für eine lineare, gesättigte oder ungesättigte $C_{12}$-$C_{18}$-Alkylgruppe, stehen.

Der Rest R2 steht für eine lineare oder verzweigte, gesättigte oder ungesättigte $C_8$-$C_{30}$-Alkylgruppe, bevorzugt für eine lineare, gesättigte oder ungesättigte $C_{11}$-$C_{21}$-Alkylgruppe, ganz besonders bevorzugt für eine lineare, gesättigte oder ungesättigte $C_{11}$-$C_{17}$-Alkylgruppe

Der Rest R3 steht für eine lineare oder verzweigte, gesättigte oder ungesättigte $C_8$-$C_{30}$-Alkylgruppe, bevorzugt für eine lineare, gesättigte oder ungesättigte $C_{11}$-$C_{21}$-Alkylgruppe, ganz besonders bevorzugt für eine lineare, gesättigte oder ungesättigte $C_{11}$-$C_{17}$-Alkylgruppe

Der Rest R4 steht für eine lineare oder verzweigte, gesättigte oder ungesättigte $C_8$-$C_{30}$-Alkylgruppe, bevorzugt für eine lineare, gesättigte oder ungesättigte $C_{12}$-$C_{18}$-Alkylgruppe.

Der Rest R5 steht für eine lineare oder verzweigte, gesättigte oder ungesättigte $C_8$-$C_{30}$-Alkylgruppe, bevorzugt für eine lineare, gesättigte oder ungesättigte $C_{12}$-$C_{18}$-Alkylgruppe.

Besonders geeignete zwitterionische Tenside der Formel (T-2) sind Alkylamidoalkylbetaine. Zu den insbesondere geeigneten amphoteren Tensiden zählen die unter der INCI-Bezeichnung bekannten Tenside Cocamidopropylbetain und der INCI Bezeichnung Cocamidopropyl Betaine.

Besonders geeignette zwitterionische Tenside der Formel (T-3) sind beispielsweise $C_{12}$-$C_{14}$-Alkyldimethylbetaine, die unter dem INCI Namen Coco-Betaine in Form des Handelsproduktes Genagen KB von der Firma Global Amines

(ehemals Clariant) bezogen werden können. Coco-Betain besitzt die CAS-Nummer 66455-29-6.

Um mit dem erfindungsgemäßen Verfahren besonders gute Entfärberesultate zu erzielen, enthält das Entfärbemittel die amphoteren bzw. zwitterionischen Tenside bevorzugt in bestimmten Mengenbereichen. Besonders gute Effekte konnten erhalten werden, wenn das Entfärbemittel - bezogen auf das Gesamtgewicht des Entfärbemittels - ein oder mehrere amphotere und/oder zwitterionische Tenside in einer Gesamtmenge von 0,5 bis 8,5 Gew.-%, bevorzugt 1,0 bis 7,5 Gew.-%, weiter bevorzugt 1,5 bis 6,5 Gew.-% und ganz besonders bevorzugt 2,0 bis 4,5 Gew.-% enthielt.

Im Rahmen einer ganz besonders bevorzugten Ausführungsform ist ein erfindungsgemäßes

Verfahren dadurch gekennzeichnet, dass das Entfärbemittel - bezogen auf das Gesamtgewicht des Entfärbemittels - ein oder mehrere amphotere und/oder zwitterionische Tenside in einer Gesamtmenge von 0,5 bis 8,5 Gew.-%, bevorzugt 1,0 bis 7,5 Gew.-%, weiter bevorzugt 1,5 bis 6,5 Gew.-% und ganz besonders bevorzugt 2,0 bis 4,5 Gew.-% enthält.

Auftrag des Entfärbemittels

Innerhalb des erfindungsgemäßen Verfahrens wird das Entfärbemittel auf das gefärbte Keratinmaterial aufgetragen und nach einer Einwirkzeit wieder abgespült.

Da das Entfärbemittel auf das gefärbte Haar aufgetragen wird, muss das Entfärbemittel nach der Anwendung des zuvor beschriebenen Färbemittels auf das Keratinmaterial appliziert werden.

Anders ausgedrückt wird das Entfärbemittel auf dem Keratinmaterial angwendet, nachdem das Färbemittel ausgespült und das Keratinmaterial bevorzugt zur genauen Bestimmung des Farbergebnisses getrocknet wurde.

Der genaue Zeitpunkt der Anwendung des Entfärbemittels bestimmt sich hierbei nach dem Wunsch des Anwenders, die unerwünschte oder nicht mehr benötigte Färbung wieder zu entfernen. So kann beispielsweise das Entfärbemittel 12 bis 24 Stunden nach Anwendung des Färbemittels auf das gefärbte Keratinmaterial appliziert werden. Im Rahmen einer weiteren Ausführungsform kann der Anwender die gefärbten Keratinmatieralien, insbesondere die Haare, aber auch über einen Zeitraum von mehreren Tagen bis Wochen tragen, bis er beschließt, die Färbung wieder zu verändern bzw. seine Ursprungshaarfarbe wieder zurück haben möchte.

weitere optionale Inhaltsstoffe im Entfärbemittel

Zusätzlich zu den bereits beschriebenen Bestandteilen kann das Entfärbemittel auch noch weitere optionale Inhaltsstoffe enthalten, wie beispielsweise Lösungsmittel, anionische, nichtionische, zwitterionische und/oder kationische Polymere; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; sowie Treibmittel wie Propan-Butan-Gemische, $N_2O$, Dimethylether, $CO_2$ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Zubereitungen bevorzugt in Mengen von jeweils 0,0001 bis 25 Gew.-%, insbesondere von 0,0005 bis 15 Gew.-%, bezogen auf das Gesamtgewicht des jeweiligen Mittels, eingesetzt.

Anwendung des Entfärbemittels

Im Rahmen des erfindungsgemäßen Verfahrens wird das zuvor beschriebene Entfärbemittel auf das gefärbte Keratinmaterial aufgetragen und nach einer Einwirkzeit wieder abgespült.

Die Anwendung kann beispielsweise mit der (behandschuten) Hand oder aber mit Hilfe eines Applikators, wie beispielsweise eines Pinsels oder einer Aplicette, oder aber auch einer Bürste, oder eines Kamms erfolgen.

Abhängig davon, ob der Anwender eine vollständige Entfärbung wünscht, oder ob nur bestimmte Partien bwz. Strähnen entfärbt werden sollen, kann das Entfärbemittel entweder auf dem gesamten keratinischen Material (wie beispielsweise dem gesamten Kopfhaar) oder aber auf bestimmte Teile oder entsprechende Strähnen des Keratinmaterials oder der Keratinfasern aufgetragen werden.

Nach dem Applizieren bzw. Auftragen wird das Entfärbemittel für einen bestimmten Zeitraum auf das Keratinmaterial einwirken gelassen. Beispielsweise kann eine Einwirkzeit von 5 bis 60 Minuten, bevorzugt von 5 bis 30 Minuten, weiter bevorzugt von 5 bis 15 Minuten und ganz besonders bevorzugt von 5 bis 10 Minuten gewählt werden. Nach dieser Einwirkzeit wird das Entfärbemittel wider mit Wasser ausgespült

Im Rahmen einer weiteren bevorzugten Ausführungsform ist ein erfindungsgemäßes Verfahren dadurch gekennzeichnet, dass das Entfärbemittel auf das gefärbte Keratinmaterial aufgetragen und nach einer Einwirkzeit von 5 bis 60 Minuten, bevorzugt von 5 bis 30 Minuten, weiter bevorzugt von 5 bis 15 Minuten und ganz besonders bevorzugt von 5 bis 10 Minuten wieder abgespült wird.

Das Einwirken des Entfärbemittels auf das Keratinmaterial kann bei Raumtemperatur bzw. bei der Körpertemperatur vorgenommen werden. Zur Unterstützung oder Beschleungung des Farbabzug kann das mit dem Entfärbemittel beaufschlagte Keratinmaterial jedoch auch erhöhten Temperaturen ausgesetzt werden. Es ist erfindungsgemäß, wenn das Entfärbemittel auf das gefärbte Keratinmaterial aufgetragen wir das Keratinmaterial während des Einwirkens des Entfärbemittels auf eine Temperatur von 25 bis 70 °, bevorzugt von 25 bis 60 °C, weiter bevorzugt von 30 bis 55 °C und ganz besonders bevorzugt von 40 bis 55 °C erhitzt wird.

Im Rahmen einer weiteren b Ausführungsform ist ein erfindungsgemäßes Verfahren dadurch gekennzeichnet, dass

- das Entfärbemittel auf das gefärbte Keratinmaterial aufgetragen wird
- das Keratinmaterial während des Einwirkens des Entfärbemittels auf eine Temperatur von 25 bis 70 °, bevorzugt von 25 bis 60 °C, weiter bevorzugt von 30 bis 55 °C und ganz besonders bevorzugt von 40 bis 55 °C erhitzt wird, und dann
- das Entfärbemittel wieder abgespült wird.

Neben der thermischen Unterstützung des Entfärbeprozesses ist es darüberhinaus auch möglich, das mit dem Entfärbemittel beaufschlagte Keratinmaterial einer mechanischen Beanspruchung auszusetzen, um die Ablösung des bei der Färbung auf dem Keratinmaterial ausgebildeten Films zu verbessern. So kann das Keratinmaterial während des Entfärbevorgangs beispielsweise mit den Händen massiert oder mit einem Kamm oder einer Bürste gekämmte werden. Auch jede andere machanische Beanspruchung, die geeignet ist, unter Einwirkung des Entfärbemittels die Ablösung des gefärbten Films vom Keratinmaterial zu verbessern, ist denkbar und vom erfindungsgemäßen Verfahren umasst.

Im Rahmen einer weiteren bevorzugten Ausführungsform ist ein erfindungsgemäßes Verfahren dadurch gekennzeichnet, dass

- das Entfärbemittel auf das gefärbte Keratinmaterial aufgetragen wird,
- das Keratinmaterial während des Einwirkens des Entfärbemittels gekämmt, massiert, gebürstet oder auf eine andere Weise einer mechanischen Krafteinwirkung ausgesetzt wird, und dann
- das Entfärbemittel wieder abgespült wird.

Wie bereits zuvor beschrieben, kann das erfindungsgemäße Entfarbemittel angewendet werden, um Keratinmaterial, welches durch Anwendung eines Pigments, bzw. mindestens eines Pigments und mindestens eines Aminosilikons, gefärbt wurde, zu entfärben. Stellt der Anwender zum Beispiel nach der Färbung fest, dass das Farbergebnis nicht seinen Wünschen entspricht, so kann er dies zum Anlass nehmen, die Färbung durch Anwendung des Entfärbemittels wider zu entfernen.

Weiterhin kann der Anwender eine Färbung und die spätere Entfärbung auch von vorneherein planen, beispielsweise, wenn er seine Haare zu einem bestimmten Anlass färben und danach wieder entfärben möchte. Zu diesem Zweck können dem Anwender auch alle Mittel bzw. Formuleirungen, die sowohl für die Färbung als auch für die Entfärbung notwendig sind, zur Verfügung gestellt werden.

Ein zweiter Gegenstand der vorliegenden Erfindung ist daher ein Verfahren zum Färben und späteren Entfärben von menschlichen Haaren, umfassend die folgenden Schritte:

(1) Auftragen eines Färbemittels auf die Haare, wobei das Färbemittel mindestens ein aminofunktionalisiertes Silikonpolymer und mindestens ein Pigment enthält, wie sie bei der Beschreibung des ersten Erfindungsgegenstands bereits im Detail offenbart wurden,
(2) Einwirkenlassen des Färbemittels auf die Haare,
(3) Ausspülen des Färbemittels aus den Haaren,
(4) Auftragen eines Entfärbemittels auf die Haare, wobei das Entfärbemittel bei der Beschreibung des ersten

Erfindungsgegenstand im Detail offenbart wurde,
(5) Einwirkenlassen des Entfärbemittels auf die Haare,
(6) Ausspülen des Entfärbemittels aus den Haaren.

Der bevorzugte weitere Gegenstand der vorliegenden Erfindung ist ein Verfahren zum Färben und späteren Entfärben von menschlichen Haaren, umfassend die folgenden Schritte in der angegebenen Reihenfolge:

(1) Auftragen eines Färbemittels auf die Haare, wobei das Färbemittel mindestens ein aminofunktionalisiertes Silikonpolymer und mindestens ein Pigment enthält, wie sie bei der Beschreibung des ersten Erfindungsgegenstands bereits im Detail offenbart wurden,
(2) Einwirkenlassen des Färbemittels auf die Haare,
(3) Ausspülen des Färbemittels aus den Haaren,
(4) Auftragen eines Entfärbemittels auf die Haare, wobei das Entfärbemittel bei der Beschreibung des ersten Erfindungsgegenstand im Detail offenbart wurde,
(5) Einwirkenlassen des Entfärbemittels auf die Haare,
(6) Ausspülen des Entfärbemittels aus den Haaren.

Die Pigmente und die aminofunktionalisierten Silikonpolymere wurden bereits bei der Beschreibung des ersten Erfindungsgegenstands im Detail offenbart. Auch das Entfärbemittel wurde bereits bei der Beschreibung des ersten Erfindungsgegenstands im Detail offenbart.

Mehrkomponenten-Verpackungseinheit

Für den Anwender ist es besonders komfortabel, wenn ihm die entsprechenden Färbe- und Entfärbemittel in Form einer Mehrkomponenten-Verpackungseinheit zur Verfügung gestellt werden.
Ein weiterer Gegentstand der vorliegenden Erfindung ist daher eine Mehrkomponenten-Verpackungseinheite (Kit-of-parts) zur Färbung und Entfärbung von Keratinmaterial, umfassend getrennt voneinander konfektioniert:

- einen ersten Container mit einem Färbemittel, wobei das Färbemittel mindestens ein aminofunktionalisiertes Silikonpolymer und mindestens ein Pigment enthält, wie sie bei der Beschreibung des ersten Erfindungsgegenstands bereits im Detail offenbart wurden, und
- einen zweiten Container mit einem Entfärbemittel, wobei das Entfärbemittel bei der Beschreibung des ersten Erfindungsgegenstand im Detail offenbart wurde.

Betreffend die weiteren bevorzugten Ausführungsformen der erfindungsgemäßen Mehrkomponenten-Verpackungseinheit und der Verwendung gilt *mutatis mutantis* das zum erfindungsgemäßen Verfahren gesagte.

Beispiele

1. Formulierungen

Es wurden die folgenden Formulierungen hergestellt (alle Angaben, sofern nichts anderes angegeben ist, in Gew.-%)

| Färbemittel (FM) | FM (Gew.-%) |
|---|---|
| Cetylalkohol | 3,0 |
| Stearylalkohol | 3,0 |
| Ceteareth-30, (Cetearylalkohol, ethoxylated 30 EO) | 1,5 |
| Kaliumdihydrogenphosphat | 0,35 |
| Dinatriumhydrogenphosphat | 0,72 |
| Unipure Red LC3071, organisches Pigment CI 15850 | 1,0 |
| 1,2-Propandiol | 10,0 |
| Phenoxyethanol | 0,8 |

(fortgesetzt)

| Färbemittel (FM) | FM (Gew.-%) |
|---|---|
| Dow Corning 2-8566 (Siloxanes and Silicones, 3-[(2-Aminoethyl)amino]-2-methylpropyl Me, Di-Me-Siloxane | 1,0 |
| Natriumsalicylat | 0,4 |
| Wasser | ad 100 |

| Entfärbemittel (EM) (Gew.-%) | EM-1 | EM-2 | EM-3 | EM-4 |
|---|---|---|---|---|
| Natriumlaurethsulfat (C12-C14, ethoxyliert mit 2 EO) | 8,2 | 8,2 | 8,2 | 8,2 |
| Dinatriumcocoamphodiacetat | 2,0 | 2,0 | 2,0 | 2,0 |
| Calciumcitrat | 5,0 | 5,0 | --- | --- |
| Natriumsulfat | --- | --- | 5,0 | --- |
| Magnesiumsulfat | --- | --- | --- | 5,0 |
| Dimethylcocoylbetain | 1,50 | 1,50 | 1,50 | 1,50 |
| Natronlauge/Zitronensäure | ad pH 4,5 | ad pH 4,1 | ad pH 4,4 | ad pH 4,7 |
| Wasser | ad 100 | ad 100 | ad 100 | ad 100 |

| Entfärbemittel (EM) (Gew.-%) | EM-5 | EM-6 | EM-7 | EM-8 |
|---|---|---|---|---|
| Natriumlaurethsulfat (C12-C14, ethoxyliert mit 2 EO) | 8,2 | 8,2 | 8,2 | 8,2 |
| Dinatriumcocoamphodiacetat | 2,0 | 2,0 | 2,0 | 2,0 |
| Kaliumcitrat | 5,0 | --- | --- | --- |
| Ammoniumcitrat | --- | 5,0 | --- | --- |
| Magnesiumcitrat | --- | --- | 5,0 | --- |
| Natriumcitrat | --- | --- | --- | 5,0 |
| Dimethylcocoylbetain | 1,50 | 1,50 | 1,50 | 1,50 |
| Natronlauge/Zitronensäure | ad pH 4,7 | ad pH 4,5 | ad pH 4,6 | ad pH 4,6 |
| Wasser | ad 100 | ad 100 | ad 100 | ad 100 |

| Entfärbemittel (EM) (Gew.-%) | EM-9 | EM-10 | EM-11 | EM-12 |
|---|---|---|---|---|
| Natriumlaurethsulfat (C12-C14, ethoxyliert mit 2 EO) | 8,2 | 8,2 | 8,2 | 8,2 |
| Dinatriumcocoamphodiacetat | 2,0 | 2,0 | 2,0 | 2,0 |
| Natriumphosphat | 5,0 | --- | --- | --- |
| Magnesiumphosphat | --- | 5,0 | --- | --- |
| Calciumphosphat | --- | --- | 5,0 | --- |
| Natriumsalicylat | --- | --- | --- | 5,0 |
| Dimethylcocoylbetain | 1,50 | 1,50 | 1,50 | 1,50 |
| Natronlauge/Zitronensäure | ad pH 4,4 | ad pH 4,5 | ad pH 4,2 | ad pH 4,4 |
| Wasser | ad 100 | ad 100 | ad 100 | ad 100 |

2. Anwendung

Eine Haarsträhne (Kerling, Euronaturhaar weiß) wurde mit einem Farbmessgerät der Firma Datacolor, Typ

Spectraflash 450 vermessen.

Nach der Herstellung wurde das Färbemittel (FM) auf Haarsträhnen (Kerling, Euronaturhaar weiß) appliziert. Das Färbemittel wurde für drei Minuten einwirken gelassen. Im Anschluss daran wurden die Haarsträhne gründlich (1 Minute) mit Wasser ausgewaschen, getrocknet und für 24 Stunden ruhen glassen.

Eine der gefärbten Strähnen wurde erneut mit einem Farbmessgerät der Firma Datacolor, Typ Spectraflash 450 vermessen.

Das Entfärbemittel wurde jeweils auf eine gefärbte Haarsträhne aufgetragen, für 5 Minuten einmassiert und dann mit Wasser ausgespült. Die auf diese Weise entfärbten Haare wurden trocknen gelassen und dann farbmetrisch vermessen.

Der für die Beurteilung des Farberhalts herangezogene dE-Wert ergibt sich aus den am jeweiligen Strähnenteil gemessenen L*a*b*-Farbmesswerten wie folgt:

$$dE = [\ (L_i - L_0)^2 + (a_i - a_0)^2 + (b_i - b_0)]^{1/2}$$

$L_0$, $a_0$ und $b_0$ = Messwerte der ungefärbten Strähne
$L_i$, $a_i$ und $b_i$ = Messwerte der gefärbten/entfärbten Strähne

Je kleiner der dE-Wert ist, desto geringer ist der Farbabstand im Vergleich zur ungefärbten Strähne und desto besser ist die Entfärbewirkung.

| | L | a | b | dE | Entfärbeleistung |
|---|---|---|---|---|---|
| Haarsträhne Euronaturhaar weiß, uncoloriert | 73,2 | 2,6 | 22,1 | --- | --- |
| Färbung mit (FM) | 38,6 | 48,9 | 11,2 | --- | --- |
| Färbung mit (FM) und Entfärbung mit (EM-1) | 48,7 | 29,8 | 7,8 | 39,5 | mittel |
| Färbung mit (FM) und Entfärbung mit (EM-2) | 64,2 | 17,2 | 9,5 | 21,4 | gut |
| Färbung mit (FM) und Entfärbung mit (EM-3) | 53,3 | 33,0 | 8,0 | 39,2 | mittel |
| Färbung mit (FM) und Entfärbung mit (EM-4) | 57,1 | 16,0 | 3,2 | 28,3 | gut |
| Färbung mit (FM) und Entfärbung mit (EM-5) | 53,3 | 30,6 | 7,1 | 37,7 | mittel |
| Färbung mit (FM) und Entfärbung mit (EM-6) | 53,9 | 24,6 | 9,1 | 32,2 | mittel |
| Färbung mit (FM) und Entfärbung mit (EM-7) | 65,8 | 12,2 | 9,5 | 17,6 | gut |
| Färbung mit (FM) und Entfärbung mit (EM-8) | 62,6 | 14,8 | 11,2 | 19,6 | gut |
| Färbung mit (FM) und Entfärbung mit (EM-9) | 55,8 | 30,4 | 9,9 | 35,1 | mittel |
| Färbung mit (FM) und Entfärbung mit (EM-10) | 58,5 | 17,7 | 3,3 | 28,3 | gut |
| Färbung mit (FM) und Entfärbung mit (EM-11) | 65,6 | 12,1 | 10,2 | 17,1 | gut |
| Färbung mit (FM) und Entfärbung mit (EM-12) | 67,2 | 13,1 | 12,7 | 15,5 | gut |
| dE = Farbabstand zum unbehandelten Haar | | | | | |

## Patentansprüche

1. Verfahren zur Entfärbung von Keratinmaterial, welches durch Anwendung von mindestens einem aminofunktionalisierten Silikonpolymer und mindestens einem Pigment gefärbt wurde, wobei ein Entfärbemittel, welches

   (a) mindestens ein Salz eines ein- oder zweiwertigen Kations enthält, und
   (b) einen pH-Wert von 1,0 bis 4,7 besitzt,

   auf das gefärbte Keratinmaterial aufgetragen und nach einer Einwirkzeit wieder abgespült wird.

2. Verfahren nach Anspruch 1, **dadurch gekennzeichnet, dass** das Entfärbemittel auf Keratinmaterial aufgetragen wird, welches durch Anwendung von mindestens einem aminofunktionalisierten Silikonpolymer mit mindestens einer

sekundären Aminogruppe gefärbt wurde.

3. Verfahren nach einem der Ansprüche 1 bis 2, **dadurch gekennzeichnet, dass** das Entfärbemittel auf Keratinmaterial aufgetragen wird, welches durch Anwendung von mindestens einem aminofunktionalisierten Silikonpolymer gefärbt wurde, welches mindestens eine Struktureinheit der Formel (Si-Amino) umfasst,

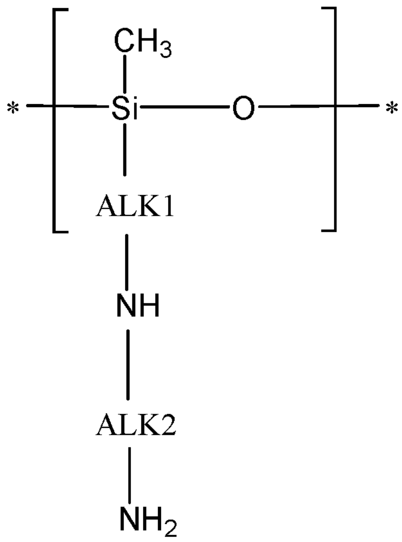

(Si-Amino)

wobei

ALK1 und ALK2 unabhängig voneinander für eine lineare oder verzweigte, zweiwertige $C_1$-$C_{20}$-Alkylengruppe stehen.

4. Verfahren nach einem der Ansprüche 1 bis 3, **dadurch gekennzeichnet, dass** das Entfärbemittel auf Keratinmaterial aufgetragen wird, welches durch Anwendung von mindestens einem aminofunktionalisierten Silikonpolymer gefärbt wurde,welches Struktureinheiten der Formel (Si-I) und der Formel (Si-II) umfasst

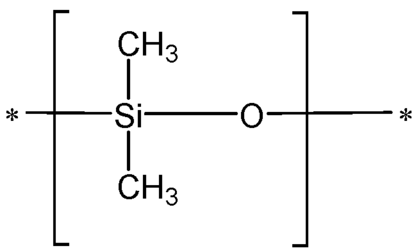

(Si-I)

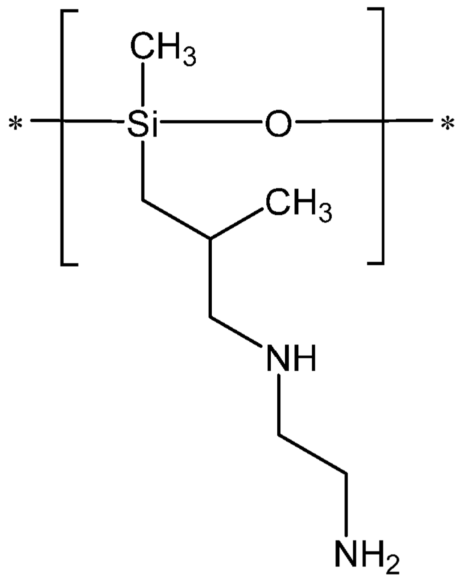

(Si-II).

5. Verfahren nach einem der Ansprüche 1 bis 4, **dadurch gekennzeichnet, dass** das Entfärbemittel auf Keratinmaterial aufgetragen wird, welches durch Anwendung von mindestens einem anorganischen Pigment gefärbt wurde, wobei das anorganische Pigment bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus

farbigen Pigmenten auf Mica- oder Glimmerbasis, die mit mindestens einem Metalloxid und/oder einem Metalloxychlorid beschichtet sind.

6. Verfahren nach einem der Ansprüche 1 bis 5, **dadurch gekennzeichnet, dass** das Entfärbemittel auf Keratinmaterial aufgetragen wird, welches durch Anwendung von mindestens einem organischen Pigment gefärbt wurde, wobei das organische Pigment bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

7. Verfahren nach einem der Ansprüche 1 bis 6, **dadurch gekennzeichnet, dass** das Entfärbemittel mindestens ein Salz eines ein- oder zweiwertigen Kations (a) enthält, das ausgewählt ist aus der Gruppe der Calciumsalze, der Magnesiumsalze, der Natriumsalze, der Kaliumsalze und der Ammoniumsalze.

8. Verfahren nach einem der Ansprüche 1 bis 7, **dadurch gekennzeichnet, dass** das Entfärbemittel mindestens ein Salz eines ein- oder zweiwertigen Kations (a) enthält, das ausgewählt ist aus der Gruppe aus

- Calciumcitrat, Magnesiumcitrat, Natriumcitrat, Ammoniumcitrat,
- Calciumsalicylat, Magnesiumsalicylat, Natriumsalicylat, Ammoniumsalicylat,
- Calciumphosphat, Magnesiumphosphat, Natriumphosphat, Ammoniumphosphat,
- Calciumsulfat, Magnesiumsulfat, Natriumsulfat, Ammoniumsulfat,
- Calciumtartrat, Magnesiumtartrat, Natriumtartrat, Ammoniumtartrat,
- Calciumlactat, Magnesiumlactat, Natriumlactat, Ammoniumlactat,
- Calciummalat, Magnesiummalat, Natriummalat, Ammoniummalat,
- Calciumsuccinat, Magnesiumsuccinat, Natriumsuccinat, Ammoniumsuccinat,
- Calciumbenzoat, Magnesiumbenzoat, Natriumbenzoat, Ammoniumbenzoat,
- Calciumfumarat, Magnesiumfumarat, Natriumfumarat, Ammoniumfumarat,
- Calciummaleinat, Magnesiummaleinat, Natriummaleinat, Ammoniummaleinat,
- Calciumacetat, Magnesiumacetat, Natriumacetat, Ammoniumacetat,
- Calciumchlorid, Magnesiumchlorid, Natriumchlorid, Ammoniumchlorid,
- Calciumbromid, Magnesiumbromid, Natriumbromid und/oder Ammoniumbromid.

9. Verfahren nach einem der Ansprüche 1 bis 8, **dadurch gekennzeichnet, dass** das Entfärbemittel - bezogen auf das Gesamtgewicht des Entfärbemittels - ein oder mehrere Salze von ein- oder zweiwertigen Kationen (a) in einer Gesamtmenge von 1,0 bis 15,0 Gew.-%, bevorzugt 1,5 bis 13,0 Gew.-%, weiter bevorzugt 3,0 bis 12,0 Gew.-% und ganz besonders bevorzugt 4,5 bis 8,0 Gew.-% enthält.

10. Verfahren nach einem der Ansprüche 1 bis 9, **dadurch gekennzeichnet, dass** das Entfärbemittel einen pH-Wert (b) von 1,5 bis 4,6, bevorzugt von 2,0 bis 4,6, weiter bevorzugt von 2,5 bis 4,6, noch weiter bevorzugt von 3,0 bis 4,6 und ganz besonders bevorzugt von 3,8 bis 4,6 besitzt.

11. Verfahren nach einem der Ansrpüche 1 bis 10, **dadurch gekennzeichnet, dass** das Entfärbemittel mindestens ein anionisches und/oder zwitterionisches Tensid enthält.

12. Verfahren nach einem der Ansprüche 1 bis 11, **dadurch gekennzeichnet, dass** das Entfärbemittel mindestens ein anionisches Tensid der Formel (T-1) enthält,

$$R_1 - \left( O - CH_2 - CH_2 \right)_x - O - SO_3M$$

(T-1),

wobei

$R_1$ für eine lineare oder verzweigte, gesättigte oder ungesättigte $C_8$-$C_{30}$-Alkylgruppe steht,
x für eine ganze Zahl von 0 bis 50 steht, und
M für ein Wasserstoffatom, für Ammonium $(NH_4)_+$ oder für ein Äquivalent eines ein oder mehrwertigen Kations steht.

**13.** Verfahren nach einem der Ansprüche 1 bis 12, **dadurch gekennzeichnet, dass** das Entfärbemittel - bezogen auf das Gesamtgewicht des Entfärbemittels - ein oder mehrere anionische Tenside in einer Gesamtmenge von 2,0 bis 18,0 Gew.-%, bevorzugt von 4,0 bis 16,0 Gew.-%, weiter bevorzugt von 6,0 bis 14,0 Gew.-% und ganz besonders bevorzugt von 8,0 bis 12,0 Gew.-% enthält.

**14.** Verfahren nach einem der Ansprüche 1 bis 13, **dadurch gekennzeichnet,dass** das Entfärbemittel mindestens ein zwitterionisches Tensid enthält, das ausgewählt ist aus den Tensiden der Formel (T-2), (T-3), (T-4) und/oder (T-5),

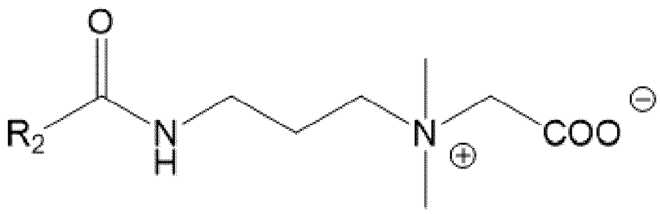

(T-2)

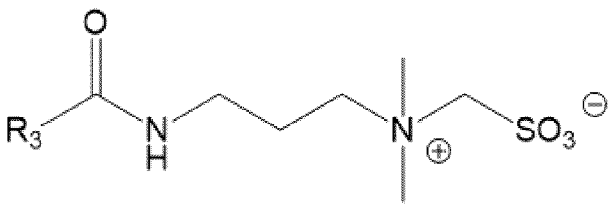

(T-3)

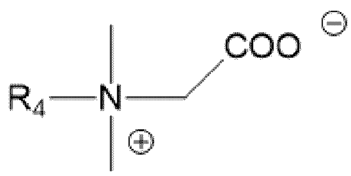

(T-4)

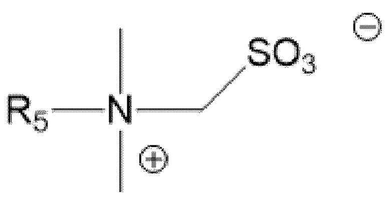

(T-5)

wobei

$R_2$, $R_3$, $R_4$, $R_5$ unabhängig voneinander für eine lineare oder verzweigte, gesättigte oder ungesättigte $C_8$-$C_{30}$-Alkylgruppe, bevorzugt für eine lineare, gesättigte oder ungesättigte $C_{12}$-$C_{18}$-Alkylgruppe, stehen.

**15.** Verfahren nach einem der Ansprüche 1 bis 14, **dadurch gekennzeichnet, dass** das Entfärbemittel - bezogen auf das

Gesamtgewicht des Entfärbemittels - ein oder mehrere amphotere und/oder zwitterionische Tenside in einer Gesamtmenge von 0,5 bis 8,5 Gew.-%, bevorzugt 1,0 bis 7,5 Gew.-%, weiter bevorzugt 1,5 bis 6,5 Gew.-% und ganz besonders bevorzugt 2,0 bis 4,5 Gew.-% enthält.

**16.** Verfahren zum Färben und späteren Entfärben von menschlichen Haaren, umfassend die folgenden Schritte:

(1) Auftragen eines Färbemittels auf die Haare, wobei das Färbemittel mindestens ein aminofunktionalisiertes Silikonpolymer und mindestens ein Pigment enthält, wie sie in den Ansprüchen 1 bis 6 definiert wurden,
(2) Einwirkenlassen des Färbemittels auf die Haare,
(3) Ausspülen des Färbemittels aus den Haaren,
(4) Auftragen eines Entfärbemittels auf die Haare, wobei das Entfärbemittel in den Ansprüchen 1 bis 15 definiert wurde,
(5) Einwirkenlassen des Entfärbemittels auf die Haare,
(6) Ausspülen des Entfärbemittels aus den Haaren.

## Claims

**1.** Process for decolorizing keratin material which has been colored by applying at least one amino-functionalized silicone polymer and at least one pigment, wherein a decolorizing agent which

(a) contains at least one salt of a monovalent or divalent cation, and
(b) has a pH of 1.0 to 4.7,

is applied to the dyed keratin material and rinsed off after a reaction time.

**2.** Process according to claim 1, **characterized in that** the decolorizing agent is applied to keratin material which has been dyed by applying at least one amino-functionalized silicone polymer with at least one secondary amino group.

**3.** Method according to one of claims 1 to 2, **characterized in that** the decolorizing agent is applied to keratin material which has been colored by the application of at least one amino-functionalized silicone polymer which comprises at least one structural unit of the formula (Si-amino)

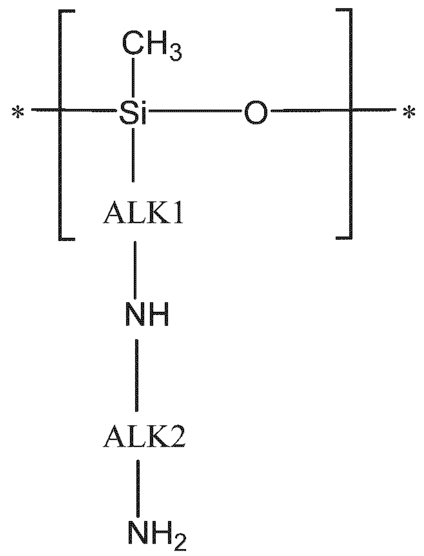

(Si-amino)

where
ALK1 and ALK2 independently represent a linear or branched, divalent $C_1$-$C_{20}$ alkylene group.

**4.** Method according to one of claims 1 to 3, **characterized in that** the decolorizing agent is applied to keratin material which has been colored by the application of at least one amino-functionalized silicone polymer which comprises structural units of the formula (Si-I) and the formula (Si-II)

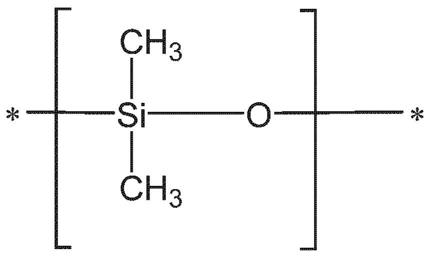

(Si-I)

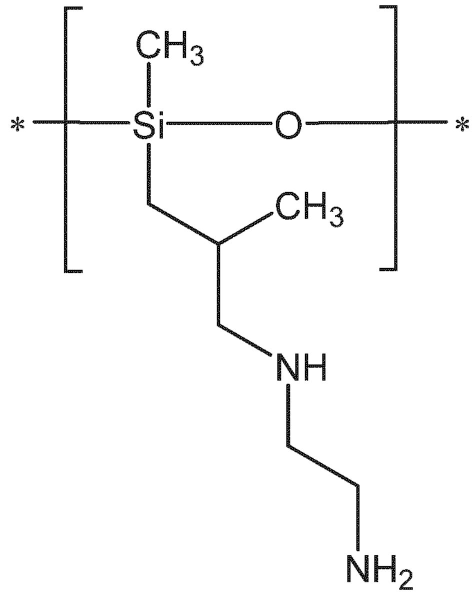

(Si-II).

5. Process according to one of claims 1 to 4, **characterized in that** the decolorizing agent is applied to keratin material which has been colored by the application of at least one inorganic pigment, the inorganic pigment being preferably selected from the group consisting of colored metal oxides, metal hydroxides, metal oxide hydrates, silicates, metal sulfides, complex metal cyanides, metal sulfates, bronze pigments and/or colored pigments based on mica or micaceous materials coated with at least one metal oxide and/or a metal oxychloride.

6. Method according to any of claims 1 to 5, **characterized in that** the decolorizing agent is applied to keratin material which has been colored by applying at least one organic pigment, the organic pigment being preferably selected from the group consisting of carmine, quinacridone, phthalocyanine, sorghum, blue pigments with the Color Index numbers CI 42090, CI 69800, CI 69825, CI 73000, CI 74100, CI 74160, yellow pigments with the Color Index numbers CI 11680, CI 11710, CI 15985, CI 19140, CI 20040, CI 21100, CI 21108, CI 47000, CI 47005, green pigments with Color Index numbers CI 61565, CI 61570, CI 74260, orange pigments with Color Index numbers CI 11725, CI 15510, CI 45370, CI 71105, red pigments with Color Index numbers CI 12085, CI 12120, CI 12370, CI 12420, CI 12490, CI 14700, CI 15525, CI 15580, CI 15620, CI 1 5630, CI 15800, CI 15850, CI 15865, CI 15880, CI 17200, CI 26100, CI 45380, CI 45410, CI 58000, CI 73360, CI 73915 and/or CI 754 70.

7. Method according to one of claims 1 to 6, **characterized in that** the decolorizing agent contains at least one salt of a monovalent or divalent cation (a) selected from the group consisting of calcium salts, magnesium salts, sodium salts, potassium salts, and ammonium salts.

8. Method according to one of claims 1 to 7, **characterized in that** the decolorizing agent contains at least one salt of a monovalent or divalent cation (a) selected from the group consisting of

- calcium citrate, magnesium citrate, sodium citrate, ammonium citrate,
- calcium salicylate, magnesium salicylate, sodium salicylate, ammonium salicylate,
- calcium phosphate, magnesium phosphate, sodium phosphate, ammonium phosphate,
- calcium sulfate, magnesium sulfate, sodium sulfate, ammonium sulfate,
- calcium tartrate, magnesium tartrate, sodium tartrate, ammonium tartrate,
- calcium lactate, magnesium lactate, sodium lactate, ammonium lactate,
- calcium malate, magnesium malate, sodium malate, ammonium malate,
- calcium succinate, magnesium succinate, sodium succinate, ammonium succinate,
- calcium benzoate, magnesium benzoate, sodium benzoate, ammonium benzoate,
- calcium fumarate, magnesium fumarate, sodium fumarate, ammonium fumarate,
- calcium maleate, magnesium maleate, sodium maleate, ammonium maleate,
- calcium acetate, magnesium acetate, sodium acetate, ammonium acetate,

- calcium chloride, magnesium chloride, sodium chloride, ammonium chloride,
- calcium bromide, magnesium bromide, sodium bromide and/or ammonium bromide.

**9.** Method according to one of claims 1 to 8, **characterized in that** the decolorizing agent contains, based on the total weight of the decolorizing agent, one or more salts of monovalent or divalent cations (a) in a total amount of 1.0 to 15.0 wt. -%, preferably 1.5 to 13.0 wt.%, more preferably 3.0 to 12.0 wt.% and most preferably 4.5 to 8.0 wt.%.

**10.** Process according to one of claims 1 to 9, **characterized in that** that the decolorizing agent has a pH value (b) of 1.5 to 4.6, preferably of 2.0 to 4.6, more preferably of 2.5 to 4.6, even more preferably of 3.0 to 4.6, and most preferably of 3.8 to 4.6.

**11.** Method according to one of claims 1 to 10, **characterized in that** the decolorizing agent contains at least one anionic and/or zwitterionic surfactant.

**12.** Method according to one of claims 1 to 11, **characterized in that** the decolorizing agent contains at least one anionic surfactant of formula (T-1),

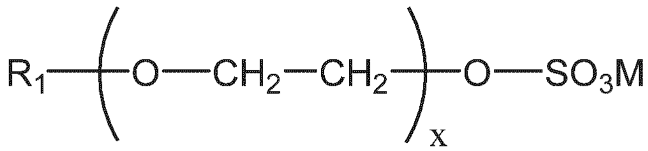

(T-1),

where

$R_1$ stands for a linear or branched, saturated or unsaturated $C_8$-$C_{30}$ alkyl group,
x stands for an integer from 0 to 50, and
M stands for a hydrogen atom, for ammonium $(NH_4)_+$ or for an equivalent of a monovalent or polyvalent cation.

**13.** Method according to any of claims 1 to 12, **characterized in that** the decolorizing agent contains, based on the total weight of the decolorizing agent, one or more anionic surfactants in a total amount of 2.0 to 18.0 wt.%, preferably 4.0 to 16.0 wt. -%, more preferably from 4.0 to 16.0 wt.% and most preferably from 8.0 to 12.0 wt.%.

**14.** Method according to one of claims 1 to 13, **characterized in that** the decolorizing agent contains at least one zwitterionic surfactant selected from the surfactants of formula (T-2), (T-3), (T-4) and/or (T-5),

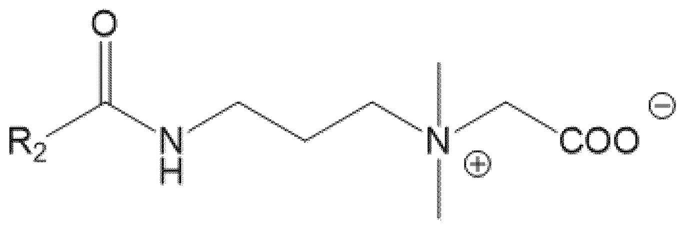

(T-2)

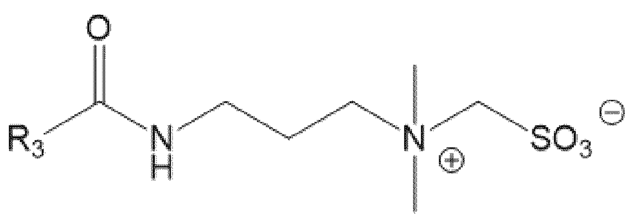

(T-3)

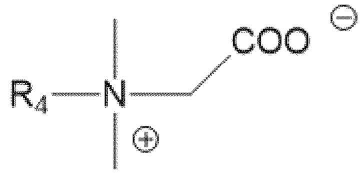

(T-4)

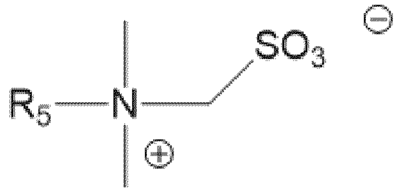

(T-5)

where
$R_2$, $R_3$, $R_4$, $R_5$ independently of one another represent a linear or branched, saturated or unsaturated $C_8$-$C_{30}$ alkyl group, preferably a linear, saturated or unsaturated $C_{12}$-$C_{18}$ alkyl group.

**15.** Method according to one of claims 1 to 14, **characterized in that** the decolorizing agent contains, based on the total weight of the decolorizing agent, one or more amphoteric and/or zwitterionic surfactants in a total amount of 0.5 to 8.5 wt.%, preferably 1.0 to 7.5 wt. -%, more preferably 1.5 to 6.5 wt.%, and most preferably 2.0 to 4.5 wt.%.

**16.** Process for dyeing and subsequent decolorizing human hair, comprising the following steps:

(1) applying a dyeing agent to the hair, wherein the dyeing agent contains at least one aminofunctionalized silicone polymer and at least one pigment as defined in claims 1 to 6,
(2) allowing the dyeing agent to act on the hair,
(3) rinsing the dyeing agent out of the hair,
(4) applying a decolorant to the hair, wherein the decolorant is defined in claims 1 to 15,
(5) allowing the decolorant to act on the hair,
(6) rinsing the decolorant from the hair.

**Revendications**

**1.** Procédé de décoloration de matière kératinique qui a été colorée par application d'au moins un polymère de silicone à fonction amino et d'au moins un pigment, dans lequel un agent décolorant qui

(a) contient au moins un sel d'un cation monovalent ou divalent, et
(b) a un pH de 1,0 à 4,7,

est appliqué sur la matière kératinique colorée et rincé après un temps d'action.

**2.** Procédé selon la revendication 1, **caractérisé en ce que** l'agent décolorant est appliqué sur une matière kératinique qui a été colorée par application d'au moins un polymère de silicone à fonction amino avec au moins un groupe amino secondaire.

**3.** Procédé selon l'une des revendications 1 à 2, **caractérisé en ce que** l'agent décolorant est appliqué sur une matière kératinique qui a été colorée par l'application d'au moins un polymère de silicone à fonction amino comprenant au moins une unité structurale de formule (Si-amino)

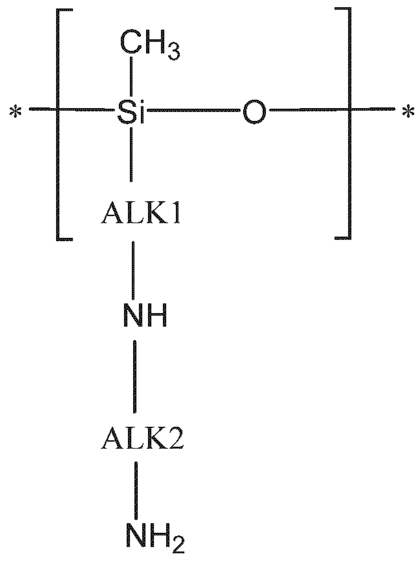

(Si-amino)

ALK1 et ALK2 représentent indépendamment l'un de l'autre un groupe alkylène $C_1$-$C_{20}$ divalent linéaire ou ramifié.

4. Procédé selon l'une des revendications 1 à 3, **caractérisé en ce que** l'agent décolorant est appliqué sur une matière kératinique qui a été colorée par l'application d'au moins un polymère de silicone à fonction amino, qui comprend des unités structurales de formule (Si-I) et de formule (Si-II)

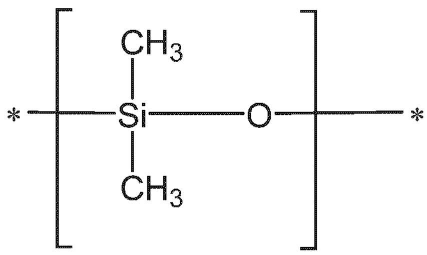

(Si-I)

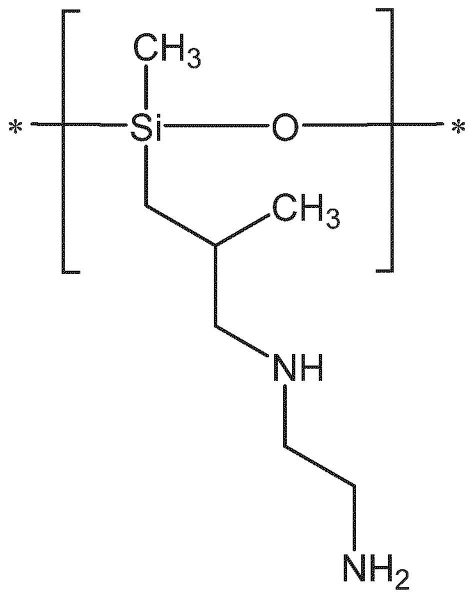

(Si-II).

5. Procédé selon l'une des revendications 1 à 4, **caractérisé en ce que** l'agent décolorant est appliqué sur une matière kératinique qui a été colorée par l'application d'au moins un pigment inorganique, le pigment inorganique étant de préférence choisi dans le groupe des oxydes métalliques colorés, des hydroxydes métalliques, des hydrates d'oxydes métalliques, des silicates, des sulfures métalliques, des cyanures métalliques complexes, des sulfates métalliques, pigments de bronze et/ou de pigments colorés à base de mica ou de mica recouverts d'au moins un oxyde métallique et/ou un oxychlorure métallique.

6. Procédé selon l'une des revendications 1 à 5, **caractérisé en ce que** l'agent décolorant est appliqué sur une matière kératinique qui a été colorée par l'application d'au moins un pigment organique, le pigment organique étant de

préférence choisi parmi le groupe comprenant le carmin, la quinacridone, la phtalocyanine, le sorgho, les pigments bleus ayant les numéros d'index de couleur CI 42090, CI 69800, CI 69825, CI 73000, CI 74100, CI 74160, les pigments jaunes ayant les numéros d'index de couleur CI 11680, CI 11710, CI 15985, CI 19140, CI 20040, CI 21100, CI 21108, CI 47000, CI 47005, pigments verts portant les numéros d'index de couleur CI 61565, CI 61570, CI 74260, pigments orange portant les numéros d'index de couleur CI 11725, CI 15510, CI 45370, CI 71105, pigments rouges portant les numéros d'index de couleur CI 12085, CI 12120, CI 12370, CI 12420, CI 12490, CI 14700, CI 15525, CI 15580, CI 15620, CI 1 5630, CI 15800, CI 15850, CI 15865, CI 15880, CI 17200, CI 26100, CI 45380, CI 45410, CI 58000, CI 73360, CI 73915 et/ou CI 754 70.

**7.** Procédé selon l'une des revendications 1 à 6, **caractérisé en ce que** l'agent décolorant contient au moins un sel d'un cation monovalent ou divalent (a) choisi dans le groupe des sels de calcium, des sels de magnésium, des sels de sodium, des sels de potassium et des sels d'ammonium.

**8.** Procédé selon l'une des revendications 1 à 7, **caractérisé en ce que** l'agent décolorant contient au moins un sel d'un cation monovalent ou divalent (a) choisi dans le groupe constitué par

- le citrate de calcium, le citrate de magnésium, le citrate de sodium, le citrate d'ammonium,
- le salicylate de calcium, le salicylate de magnésium, le salicylate de sodium, le salicylate d'ammonium,
- le phosphate de calcium, le phosphate de magnésium, le phosphate de sodium, le phosphate d'ammonium,
- le sulfate de calcium, le sulfate de magnésium, le sulfate de sodium, le sulfate d'ammonium,
- tartrate de calcium, tartrate de magnésium, tartrate de sodium, tartrate d'ammonium,
- lactate de calcium, lactate de magnésium, lactate de sodium, lactate d'ammonium,
- malate de calcium, malate de magnésium, malate de sodium, malate d'ammonium,
- succinate de calcium, succinate de magnésium, succinate de sodium, succinate d'ammonium,
- benzoate de calcium, benzoate de magnésium, benzoate de sodium, benzoate d'ammonium,
- fumarate de calcium, fumarate de magnésium, fumarate de sodium, fumarate d'ammonium,
- maléate de calcium, maléate de magnésium, maléate de sodium, maléate d'ammonium,
- acétate de calcium, acétate de magnésium, acétate de sodium, acétate d'ammonium,
- chlorure de calcium, chlorure de magnésium, chlorure de sodium, chlorure d'ammonium,
- bromure de calcium, bromure de magnésium, bromure de sodium et/ou bromure d'ammonium.

**9.** Procédé selon l'une des revendications 1 à 8, **caractérisé en ce que** l'agent décolorant contient, par rapport au poids total de l'agent décolorant, un ou plusieurs sels de cations monovalents ou divalents (a) en une quantité totale de 1,0 à 15,0 % en poids - %, de préférence de 1,5 à 13,0 % en poids, de préférence de 3,0 à 12,0 % en poids et de manière particulièrement préférée de 4,5 à 8,0 % en poids.

**10.** Procédé selon l'une des revendications 1 à 9, **caractérisé en ce que** que l'agent décolorant a un pH (b) de 1,5 à 4,6, de préférence de 2,0 à 4,6, de préférence encore de 2,5 à 4,6, de préférence encore plus de 3,0 à 4,6 et de préférence tout particulièrement de 3,8 à 4,6.

**11.** Procédé selon l'une des revendications 1 à 10, **caractérisé en ce que** l'agent décolorant contient au moins un tensioactif anionique et/ou zwitterionique.

**12.** Procédé selon l'une des revendications 1 à 11, **caractérisé en ce que** l'agent décolorant contient au moins un tensioactif anionique de formule (T-1)

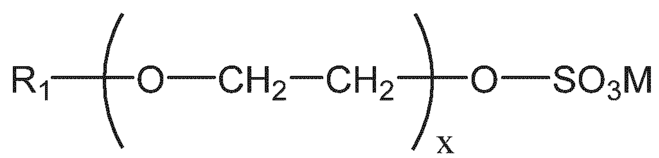

(T-1),

dans laquelle

$R_1$ représente un groupe alkyle en $C_8$ à $C_{30}$ linéaire ou ramifié, saturé ou insaturé,

x représente un nombre entier de 0 à 50, et
M représente un atome d'hydrogène, un groupe ammonium $(NH_4)_+$ ou un équivalent d'un cation mono- ou polyvalent.

**13.** Procédé selon l'une des revendications 1 à 12, **caractérisé en ce que** l'agent décolorant contient, par rapport au poids total de l'agent décolorant, un ou plusieurs tensioactifs anioniques en une quantité totale de 2,0 à 18,0 % en poids, de préférence de 4,0 à 16,0 % en poids %, de préférence de 4,0 à 16,0 % en poids et de manière particulièrement préférée de 8,0 à 12,0 % en poids.

**14.** Procédé selon l'une des revendications 1 à 13, **caractérisé en ce que** l'agent décolorant contient au moins un tensioactif zwitterionique choisi parmi les tensioactifs de formule (T-2), (T-3), (T-4) et/ou (T-5),

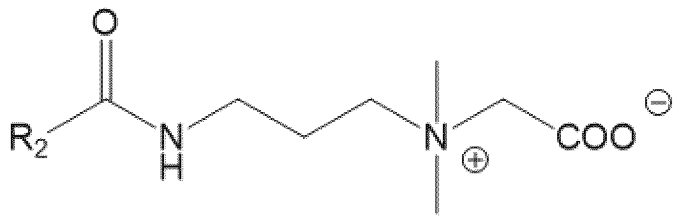

(T-2)

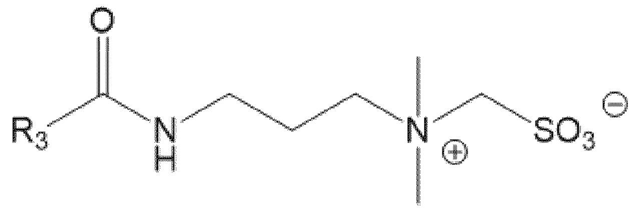

(T-3)

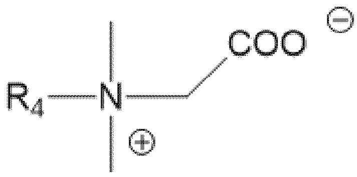

(T-4)

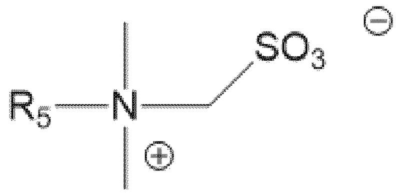

(T-5)

dans lesquelles
$R_2$, $R_3$, $R_4$, $R_5$ représentent indépendamment les uns des autres un groupe alkyle en $C_8$-$C_{30}$ linéaire ou ramifié, saturé ou insaturé, de préférence un groupe alkyle en $C_{12}$-$C_{18}$ linéaire, saturé ou insaturé.

**15.** Procédé selon l'une des revendications 1 à 14, **caractérisé en ce que** l'agent décolorant contient, par rapport au poids total de l'agent décolorant, un ou plusieurs tensioactifs amphotères et/ou zwitterioniques en une quantité totale de 0,5 à 8,5 % en poids, de préférence de 1,0 à 7,5 % en poids , de préférence de 1,5 à 7,5 % en poids et de manière particulièrement préférée de 2,0 à 4,5 % en poids.

**16.** Procédé pour colorer puis décolorer les cheveux humains, comprenant les étapes suivantes

(1) l'application d'un colorant sur les cheveux, le colorant contenant au moins un polymère de silicone à fonction amino et au moins un pigment tels que définis dans les revendications 1 à 6,
(2) l'action du colorant sur les cheveux,
(3) le rinçage du colorant des cheveux,
(4) application d'un décolorant sur les cheveux, le décolorant étant défini dans les revendications 1 à 15,
(5) action du décolorant sur les cheveux,
(6) rinçage du décolorant des cheveux.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- DE 102016209980

### In der Beschreibung aufgeführte Nicht-Patentliteratur

- *CHEMICAL ABSTRACTS*, 106842-44-8
- *CHEMICAL ABSTRACTS*, 813-94-5
- *CHEMICAL ABSTRACTS*, 5785-44-4
- *CHEMICAL ABSTRACTS*, 7779-25-1
- *CHEMICAL ABSTRACTS*, 68-04-2
- *CHEMICAL ABSTRACTS*, 54-21-7
- *CHEMICAL ABSTRACTS*, 7758-87-4
- *CHEMICAL ABSTRACTS*, 7601-54-9
- *CHEMICAL ABSTRACTS*, 15819-50-8
- *CHEMICAL ABSTRACTS*, 10101-89-0
- *CHEMICAL ABSTRACTS*, 7778-18-9
- *CHEMICAL ABSTRACTS*, 10034-76-1
- *CHEMICAL ABSTRACTS*, 10101-41-4
- *CHEMICAL ABSTRACTS*, 13397-24-5
- *CHEMICAL ABSTRACTS*, 7487-88-9
- *CHEMICAL ABSTRACTS*, 10034-99-8
- *CHEMICAL ABSTRACTS*, 3164-34-9
- *CHEMICAL ABSTRACTS*, 5892-21-7
- *CHEMICAL ABSTRACTS*, 110720-66-6
- *CHEMICAL ABSTRACTS*, 868-18-8
- *CHEMICAL ABSTRACTS*, 6106-24-7
- *CHEMICAL ABSTRACTS*, 109175-69-1
- *CHEMICAL ABSTRACTS*, 22476-07-9
- *CHEMICAL ABSTRACTS*, 4504-50-1
- *CHEMICAL ABSTRACTS*, 51307-92-7
- *CHEMICAL ABSTRACTS*, 814-80-2
- *CHEMICAL ABSTRACTS*, 72-17-3
- *CHEMICAL ABSTRACTS*, 17482-42-7
- *CHEMICAL ABSTRACTS*, 869-06-7
- *CHEMICAL ABSTRACTS*, 6150-86-3
- *CHEMICAL ABSTRACTS*, 676-46-0
- *CHEMICAL ABSTRACTS*, 22798-10-3
- *CHEMICAL ABSTRACTS*, 138-09-0
- *CHEMICAL ABSTRACTS*, 207511-06-6
- *CHEMICAL ABSTRACTS*, 7718-51-6
- *CHEMICAL ABSTRACTS*, 19855-56-2
- *CHEMICAL ABSTRACTS*, 10043-52-4
- *CHEMICAL ABSTRACTS*, 13477-29-7
- *CHEMICAL ABSTRACTS*, 10035-04-8
- *CHEMICAL ABSTRACTS*, 25094-02-4
- *CHEMICAL ABSTRACTS*, 7774-34-7
- *CHEMICAL ABSTRACTS*, 22691-02-7
- *CHEMICAL ABSTRACTS*, 7789-41-5
- *CHEMICAL ABSTRACTS*, 71626-99-8
- *CHEMICAL ABSTRACTS*, 22208-73-7
- *CHEMICAL ABSTRACTS*, 13477-28-6
- *CHEMICAL ABSTRACTS*, 7786-30-3
- *CHEMICAL ABSTRACTS*, 7791-18-6
- *CHEMICAL ABSTRACTS*, 7789-48-2
- *CHEMICAL ABSTRACTS*, 13446-53-2
- *CHEMICAL ABSTRACTS*, 68891-38-3
- *CHEMICAL ABSTRACTS*, 66455-29-6